# FASCICULE DE BREVET EUROPEEN

(11) **EP 0 946 482 B1**
(45) Date de publication et mention de la délivrance du brevet: **24.07.2002**
(21) Numéro de dépôt: 97947095.2
(22) Date de dépôt: 19.11.1997
(51) Int. Cl.: C07C 45/67, C07C 45/38, C07C 51/255, C07C 41/30, C07C 37/20, C07C 47/575, C07C 47/58

(54) **PROCEDE DE PREPARATION D'UN 4-HYDROXYBENZALDEHYDE ET DERIVES**
VERFAHREN ZUR HERSTELLUNG EINES 4-HYDROXYBENZALDEHYDS UND DERIVATEN
METHOD FOR PREPARING A 4-HYDROXYBENZALDEHYDE AND DERIVATIVES

(30) Priorité: 22.11.1996 FR 9614275
(43) Date de publication de la demande: 06.10.1999
(73) Titulaire: RHODIA CHIMIE, 92408 Courbevoie Cédex (FR)
(72) Inventeur: METIVIER, Pascal, F-69110 Sainte Foy lès Lyon (FR)
(74) Mandataire: Dutruc-Rosset, Marie-Claude
(86) Numéro de dépôt international: FR9702085
(87) Numéro de publication internationale: WO9822419

(56) Documents cités:
- WO-A-96/26175
- WO-A-96/37454
- DE-C- 72 600
- FR-A- 2 305 420
- GB-A- 774 696
- US-A- 4 351 962

## Description

La présente invention a pour objet un procédé de préparation d'un 4-hydroxybenzaldéhyde et dérivés.

L'invention concerne plus particulièrement la préparation du 3-méthoxy-4-hydroxybenzaldéhyde et du 3-éthoxy-4-hydroxybenzaldéhyde dénommés respectivement "vanilline" et "éthylvanilline".

Dans la demande de brevet française n°95/06186, on a décrit un procédé de préparation de 4-hydroxybenzaldéhydes et plus particulièrement de vanilline et d'éthylvanilline.

Le procédé décrit consiste à préparer un 3-carboxy-4-hydroxybenzaldéhyde puis à effectuer la décarboxylation dudit composé conduisant ainsi au 4-hydroxybenzaldéhyde.

Le 3-carboxy-4-hydroxybenzaldéhyde est préparé selon FR n°95/06186, à partir de l'un des composés et leurs mélanges répondant plus particulièrement aux formules suivantes (IIa), (IIb), (IIc) et (IId) données ci-après : 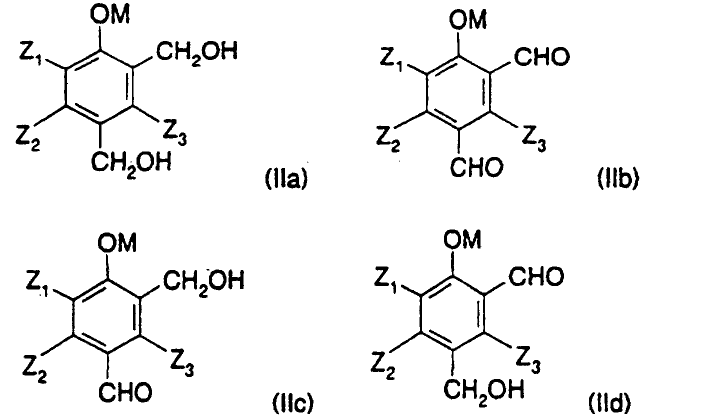 dans lesdites formules :
- M représente un atome d'hydrogène et/ou un cation métallique du groupe (la) ou (IIa) ou un cation ammonium,
- Z₁, Z₂ et Z₃, identiques ou différents, représentent un atome d'hydrogène, un radical alkyle, alcényle, alkoxy, hydroxyalkyle, alkoxyalkyle, cycloalkyle, aryle, un groupe hydroxyle, un groupe nitro, un atome d'halogène, un groupe trifluorométhyle.

Selon le procédé breveté, on part d'un composé phénolique bi-fonctionnel, portant sur le noyau aromatique, deux groupes fonctionnels en ortho et para qui peuvent être un groupe - CHO et/ou un groupe - CH₂OH.

On effectue d'abord une oxydation sélective du groupe situé en position ortho en groupe carboxylique ; le groupe situé en position para étant au plus oxydé en groupe formyle. Après élimination du groupe carboxylique situé en position ortho, on obtient un 4-hydroxybenzaldéhyde.

Ainsi, on prépare avantageusement la vanilline et l'éthylvanilline selon un procédé sélectif mais aussi très compétitif d'un point de vue industriel car il fait appel à des réactifs peu onéreux.

Toutefois, il est difficile avec ce procédé d'obtenir un rendement réactionnel (exprimé par rapport au phénol de départ) supérieur à 70 % car l'obtention du composé phénolique bi-fonctionnel avec un rendement élevé s'accompagne de celle d'un sous-produit à savoir, un bis-arylméthane.

Poursuivant ses recherches, la demanderesse a trouvé selon la demande française n°96/12479 qu'il était possible de préparer un 4-hydroxybenzaldéhyde en partant d'un mélange de composés phénoliques monosubstitués, l'un (A) porteur d'un groupe formyle ou hydroxyméthyle en position 2, et l'autre (B) porteur d'un groupe formyle ou hydroxyméthyle en position 4 et en oxydant sélectivement le groupe formyle ou hydroxyméthyle en position 2 du composé (A) en groupe carboxylique, et éventuellement d'un groupe hydroxyméthyle du composé (B) en position 4 en groupe formyle, conduisant ainsi à un mélange d'un acide 2-hydroxybenzoïque et d'un 4-hydroxybenzaldéhyde à partir duquel ce dernier est séparé.

Ainsi, le mélange de composés phénoliques mis en oeuvre répond plus particulièrement à la formule générale (II) : 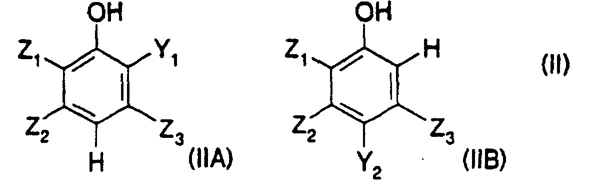 dans lesdites formules (IIA) et (IIB) :
- Y₁ et Y₂, identiques ou différents, représentent l'un des groupes suivants :
   . un groupe CHO,
   . un groupe - CH₂OH,
- Z₁, Z₂ et Z₃, identiques ou différents, représentent un atome d'hydrogène, un radical alkyle, alcényle, alkoxy, hydroxyalkyle, alkoxyalkyle, cycloalkyle, aryle, un groupe hydroxyle, un groupe nitro, un atome d'halogène, un groupe trifluorométhyle.

L'inconvénient dont souffre ce procédé est que pour obtenir les composés de formule (IIA) ou (IIB), par hydroxyméthylation d'un phénol, il importe de travailler avec un faible taux de conversion du phénol de départ ce qui induit une faible productivité.

Afin de disposer d'un procédé économiquement très intéressant qui minimise les sous-produits et permet d'obtenir une bonne productivité de l'appareillage, il était donc souhaitable de perfectionner les procédés existants.

Ainsi, il a été trouvé et c'est ce qui constitue l'objet de la présente invention, un procédé de préparation d'un 4-hydroxybenzaldéhyde et dérivés caractérisé par le fait qui consiste à oxyder sélectivement le groupe en position 2 par rapport au groupe hydroxyle, en groupe carboxylique, présent dans les composés phénoliques d'un mélange comprenant au moins :
- un composé phénolique (A) porteur de groupes formyle et/ou hydroxyméthyle en position 2 et 4,
- un composé phénolique (B) porteur d'un groupe formyle ou hydroxyméthyle en position 4,
- un composé phénolique (C) porteur d'un groupe formyle ou hydroxyméthyle en position 2,
conduisant ainsi à un mélange comprenant un 3-carboxy-4-hydroxybenzaldéhyde, un 4-hydroxybenzaldéhyde, un acide 2-hydroxybenzoique, qui est soumis ensuite à une opération de décarboxylation permettant ainsi d'obtenir le 4-hydroxybenzaldéhyde et un phénol qui peut être éventuellement recyclé.

Un autre objet de l'invention est le mélange de départ de composés phénoliques revendiqué en tant que tel et le mélange obtenu après oxydation.

Enfin, d'autres objets de l'invention sont les procédés de préparation desdits mélanges.

Conformément au procédé de l'invention, il a été trouvé qu'en partant d'un mélange de composés de départ tels que définis, il était possible d'effectuer simultanément une oxydation intramoléculaire (A) et intermoléculaire (B + C) puisque l'oxydation en groupe carboxylique a lieu préférentiellement sur le groupe hydroxyméthyle ou formyle substitué en position ortho.

Le procédé de l'invention comprend donc une étape d'oxydation et une étape de décarboxylation du 3-carboxy-4-hydroxybenzaldéhyde en 4-hydroxybenzaldéhyde et de l'acide 2-hydroxybenzoïque permettant d'aboutir au composé phénolique de départ qui peut être alors recyclé ; le 4-hydroxybenzaldéhyde étant alors récupéré d'une manière classique.

Les substrats de départ intervenant dans le procédé de l'invention sont des mélanges de composés phénoliques, l'un (A) porteur de groupes formyle et/ou hydroxyméthyle en position 2 et 4, le deuxième (B) porteur d'un groupe formyle ou hydroxyméthyle en position 4 et le dernier (C), en position 2.

Par "composé phénolique", on entend tout composé aromatique, dont le noyau aromatique est porteur d'un groupe hydroxyle.

Dans l'exposé qui suit de la présente invention, on entend par "aromatique", la notion classique d'aromaticité telle que définie dans la littérature, notamment par Jerry MARCH, Advanced Organic Chemistry, 4^{ème} édition, John Wiley and Sons, 1992, pp. 40 et suivantes.

Ainsi, on met en oeuvre un mélange (II) de composés phénoliques répondant plus particulièrement aux formules suivantes : 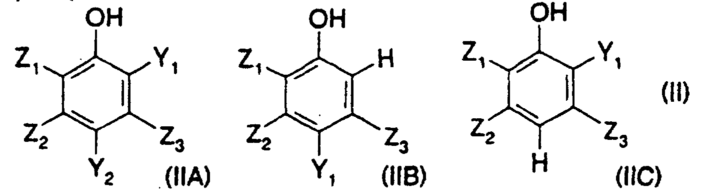 dans lesdites formules (IIA) à (IIC):
- Y₁ et Y₂, identiques ou différents, représentent l'un des groupes suivants :
   . un groupe - CHO,
   . un groupe - CH₂OH,
- Z₁, Z₂ et Z₃, identiques ou différents, représentent un atome d'hydrogène, un radical alkyle, alcényle, alkoxy, hydroxyalkyle, alkoxyalkyle, cycloalkyle, aryle, un groupe hydroxyle, un groupe nitro, un atome d'halogène, un groupe trifluorométhyle.

Les composés qui conviennent particulièrement bien à la mise en oeuvre du procédé de l'invention répondent aux formules (IIA) à (IIC) dans lesquelles Z₁, Z₂ et Z₃, identiques ou différents, représentent l'un des atomes ou groupes suivants :
. un atome d'hydrogène,
. un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
. un radical alcényle, linéaire ou ramifié, ayant de 2 à 12 atomes de carbone, de préférence de 2 à 4 atomes de carbone, tel que vinyle, allyle,
. un radical alkoxy linéaire ou ramifié ayant de 1 à 12 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un radical phényle,
. un atome d'halogène, de préférence un atome de fluor, chlore ou brome.

La présente invention n'exclut pas la présence sur le cycle aromatique de substituants d'une nature différente dans la mesure où ils n'interfèrent pas avec les réactions du procédé de l'invention.

La présente invention s'applique préférentiellement aux composés de formule (IIA) à (IIC) dans lesquelles Z₁ représente un atome d'hydrogène ou un radical alkyle ou alkoxy, linéaire ou ramifié, ayant de 1 à 6 atomes de carbone, de préférence, 1 à 4 atomes de carbone ; Z₂ et Z₃ représentant un atome d'hydrogène ; Y₁ et Y₂, identiques, représentent un groupe formyle ou un groupe hydroxyméthyle.

Comme exemples préférés de mélanges de composés phénoliques susceptibles d'être mis en oeuvre dans le procédé de l'invention, on peut citer, entre autres :
- l'o-hydroxyméthylgaïacol, le p-hydroxyméthylgaïacol et le 4,6-di(hydroxyméthyl)gaïacol,
- l'o-formylgaïacol, le p-formylgaïacol et le 4,6-diformylgaïacol,
- l'o-hydroxyméthylguétol, le p-hydroxyméthylguétol et le 4,6-di(hydroxyméthyl)guétol,
- l'o-formylguétol, le p-formylguétol et le 4,6-diformylguétol.

Conformément au procédé de l'invention, on part d'un mélange de composés phénoliques répondant préférentiellement à la formule (II).

La proportion dans le mélange de chaque composé phénolique dépend du mode de leur préparation.

On précisera que les mélanges préférentiels comprennent :
- de 30 à 70 % en poids, et plus préférentiellement de 50 à 70 % d'un composé phénolique (A),
- de 30 à 70 % en poids, et plus préférentiellement de 50 à 70 % d'un mélange de composés phénoliques (B + C).

On mentionnera à titre indicatif, que les quantités d'isomères B et C sont approximativement en quantités équimolaires dans leur mélange.

On donne ci-après, le schéma réactionnel du procédé de l'invention pour faciliter la compréhension de l'exposé de l'invention, sans pour autant lier la portée de l'invention, à celui-ci. 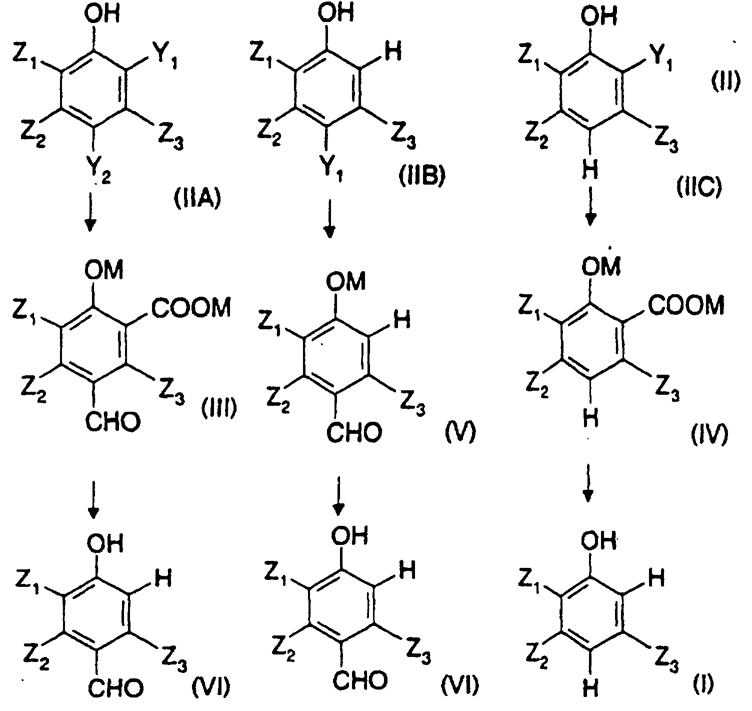 dans lesdites formules (I) à (VI) :
- Y₁ et Y₂, identiques ou différents, représentent l'un des groupes suivants :
   . un groupe - CHO,
   . un groupe - CH₂OH,
- M représente un atome d'hydrogène et/ou un cation métallique du groupe (la) ou (IIa) de la classification périodique ou un cation ammonium,
- Z₁, Z₂, Z₃, ont les significations données précédemment.

Dans le présent texte, on se réfère ci-après à la Classification périodique des éléments publiée dans le Bulletin de la Société Chimique de France, n°1 (1966).

Conformément au procédé de l'invention, on effectue une oxydation sélective du groupe Y₁ en position 2 en groupe carboxylique des composés phénoliques (A) et (C) répondant préférentiellement aux formule (IIA) et (IIC) et d'un groupe hydroxyméthyle si présent en position 4 en groupe formyle des composés phénoliques (A) et (B) répondant préférentiellement aux formules (IIA) et (IIB).

L'oxydation est réalisée par l'oxygène moléculaire ou un gaz en contenant, en présence généralement d'un catalyseur.

Un mode d'oxydation préférée consiste à oxyder un mélange de composés phénoliques de formule (II), en phase liquide, à l'aide d'oxygène moléculaire ou un gaz en contenant, en milieu aqueux renfermant un agent basique, en présence d'un catalyseur à base d'un métal M₁ choisi parmi les métaux du groupe 1b et 8 de la classification périodique des éléments comprenant éventuellement, à titre d'activateurs des métaux tels que le cadmium, le cérium, le bismuth, le plomb, l'argent, le tellure ou l'étain.

Conformément à l'invention, il a été trouvé d'une manière totalement surprenante que, si l'on augmentait la température et que l'on conduisait la réaction de préférence sous pression ou si l'on augmentait la quantité de la base présente lors de l'oxydation, on effectue une oxydation sélective du groupe Y₁ en position 2 en groupe carboxylique des composés phénoliques (A) et (C) répondant préférentiellement aux formule (IIA) et (IIC) et le groupe situé en position 4 dans les composés phénoliques (A) et (B) répondant préférentiellement aux formules (IIA) et (IIB) étant au plus oxydé en groupe formyle.

Les catalyseurs intervenant dans le procédé de l'invention sont à base d'un métal du groupe 1b et 8 de la classification périodique.

Comme exemples de catalyseurs à base d'un métal du groupe 8 de la classification périodique, on peut citer le nickel, le ruthénium, le rhodium, le palladium, l'osmium, l'iridium, le platine et leurs mélanges. Pour ce qui est des métaux du groupe 1b, on préfère faire appel au cuivre.

On utilise de préférence, des catalyseurs de platine et/ou de palladium, pris sous toutes les formes disponibles telles que par exemple : le noir de platine, le noir de palladium, l'oxyde de platine, l'oxyde de palladium ou le métal noble lui-même déposé sur des supports divers tels que le noir de carbone, le carbonate de calcium, les alumines et silices activées ou des matériaux équivalents. Des masses catalytiques à base de noir de carbone conviennent particulièrement.

La quantité de ce catalyseur à mettre en oeuvre, exprimée en poids de métal M₁ par rapport à celui du mélange de composés phénoliques de formule (II) peut varier de 0,01 à 10 % et, de préférence, de 0,04 à 2 %.

Pour plus de détails sur les catalyseurs, on peut se référer à US-A-3 673 257, FR-A-2 305 420, FR-A-2 350 323.

L'activateur peut être choisi parmi tous ceux mentionnés dans les brevets précités. De préférence, on fait appel au bismuth, au plomb et au cadmium, sous forme de métaux libres ou de cations. Dans ce dernier cas, l'anion associé n'est pas critique et on peut utiliser tous dérivés de ces métaux. De préférence, on met en oeuvre le bismuth métal ou ses dérivés.

On peut faire appel à un dérivé minéral ou organique du bismuth dans lequel l'atome de bismuth se trouve à un degré d'oxydation supérieur à zéro, par exemple égal à 2, 3, 4 ou 5. Le reste associé au bismuth n'est pas critique dès l'instant qu'il satisfait à cette condition. L'activateur peut être soluble ou insoluble dans le milieu réactionnel.

Des composés illustratifs d'activateurs qui peuvent être utilisés dans le procédé selon la présente invention sont : les oxydes de bismuth ; les hydroxydes de bismuth ; les sels d'hydracides minéraux tels que : chlorure, bromure, iodure, sulfure, séléniure, tellure de bismuth ; les sels d'oxyacides minéraux tels que : sulfite, sulfate, nitrite, nitrate, phosphite, phosphate, pyrophosphate, carbonate, perchlorate, antimoniate, arséniate, sélénite, séléniate de bismuth ; les sels d'oxyacides dérivés de métaux de transition tels que : vanadate, niobate, tantalate, chromate, molybdate, tungstate, permanganate de bismuth.

D'autres composés appropriés sont également des sels d'acides organiques aliphatiques ou aromatiques tels que : acétate, propionate, benzoate, salicylate, oxalate, tartrate, lactate, citrate de bismuth ; des phénates tels que : gallate et pyrogallate de bismuth. Ces sels et phénates peuvent être aussi des sels de bismuthyle.

Comme autres composés minéraux ou organiques, on peut utiliser des combinaisons binaires du bismuth avec des éléments tels que phosphore et arsenic ; des hétéropolyacides contenant du bismuth ainsi que leurs sels ; conviennent également les bismuthines aliphatiques et aromatiques.

A titre d'exemples spécifiques, on peut citer :
- comme oxydes : BiO ; Bi₂O₃ : Bi₂O₄ ; Bi₂O₅.
- comme hydroxydes : Bi(OH)₃,
- comme sels d'hydracides minéraux : le chlorure de bismuth BiCl₃ ; le bromure de bismuth BiBr₃ ; l'iodure de bismuth BiI₃ ; le sulfure de bismuth Bi₂S₃ ; le séléniure de bismuth Bi₂Se₃ : le tellure de bismuth Bi₂Te₃,
- comme sels d'oxyacides minéraux : le sulfite basique de bismuth Bi₂(SO₃)₃, Bi₂O₃, 5H₂O ; le sulfate neutre de bismuth Bi₂(SO₄)₃ ; le sulfate de bismuthyle (BiO)HSO₄ ; le nitrite de bismuthyle (BiO)NO₂, 0,5H₂O ; le nitrate neutre de bismuth Bi(NO₃)₃, 5H₂O ; le nitrate double de bismuth et de magnésium 2Bi(NO₃)₃, 3Mg(NO₃)₂, 24H₂O ; le nitrate de bismuthyle (BiO)NO₃ ; le phosphite de bismuth Bi₂(PO₃H)₃, 3H₂O ; le phosphate neutre de bismuth BiPO₄ ; le pyrophosphate de bismuth Bi₄(P₂O₇)₃ ; le carbonate de bismuthyle (BiO)₂CO₃, O,5H₂O ; le perchlorate neutre de bismuth Bi(ClO₄)₃, 5H₂O ; le perchlorate de bismuthyle (BiO)ClO₄ ; l'antimoniate de bismuth BiSbO₄ ; l'arséniate neutre de bismuth Bi(AsO₄)₃ ; l'arséniate de bismuthyle (BiO)AsO₄, 5H₂O ; le sélénite de bismuth Bi₂(SeO₃)₃.
- comme sels d'oxyacides dérivés de métaux de transition : le vanadate de bismuth BiVO₄ ; le niobate de bismuth BiNbO₄ : le tantalate de bismuth BiTaO₄ ; le chromate neutre de bismuth Bi₂(CrO₄) ; le dichromate de bismuthyle [(BiO)₂]₂Cr₂O₇ ; le chromate acide de bismuthyle H(BiO)CrO₄ ; le chromate double de bismuthyle et de potassium K(BiO)CrO₄ ; le molybdate de bismuth Bi₂(MoO₄)₃ ; le tungstate de bismuth Bi₂(WO₄)₃ ; le molybdate double de bismuth et de sodium NaBi(MoO₄)₂ ; le permanganate basique de bismuth Bi₂O₂(OH)MnO₄.
- comme sels d'acides organiques aliphatiques ou aromatiques : l'acétate de bismuth Bi(C₂H₃O₂)₃ ; le propionate de bismuthyle (BiO)C₃H₅O₂ ; le benzoate basique de bismuth C₆H₅CO₂Bi(OH)₂ ; le salicylate de bismuthyle C₆H₄CO₂(BiO)(OH) ; l'oxalate de bismuth (C₂O₄)₃Bi₂ ; le tartrate de bismuth Bi₂(C₄H₄O₆)₃, 6H₂O ; le lactate de bismuth (C₆H₉O₅)OBi, 7H₂O ; le citrate de bismuth C₆H₅O₇Bi.
- comme phénates : le gallate basique de bismuth C₇H₇O₇Bi ; le pyrogallate basique de bismuth C₆H₃(OH)₂(OBi)(OH).

Comme autres composés minéraux ou organiques conviennent également : le phosphure de bismuth BiP ; l'arséniure de bismuth Bi₃As₄ ; le bismuthate de sodium NaBiO₃ ; les acides bismuth-thiocyaniques H₂[Bi(BNS)₅], H₃[Bi(CNS)₆] et leurs sels de sodium et potassium ; la triméthylbismuthine Bi(CH₃)₃, la triphénylbismuthine Bi(C₆H₅)₃.

Les dérivés du bismuth qui sont utilisés de préférence pour conduire le procédé selon l'invention sont : les oxydes de bismuth ; les hydroxydes de bismuth ; les sels de bismuth ou de bismuthyle d'hydracides minéraux ; les sels de bismuth ou de bismuthyle d'oxyacides minéraux ; les sels de bismuth ou de bismuthyle d'acides organiques aliphatiques ou aromatiques ; et les phénates de bismuth ou de bismuthyle.

Un groupe d'activateurs qui conviennent particulièrement bien à la réalisation de l'invention est constitué par : les oxydes de bismuth Bi₂O₃ et Bi₂O₄ ; l'hydroxyde de bismuth Bi(OH)₃ ; le sulfate neutre de bismuth Bi₂(SO₄)₃ ; le chlorure de bismuth BiCl₃ ; le bromure de bismuth BiBr₃ ; l'iodure de bismuth Bil₃ ; le nitrate neutre de bismuth Bi(NO₃)₃, 5H₂O ; le nitrate de bismuthyle BiO(NO₃) ; le carbonate de bismuthyle (BiO)₂CO₃, O,5H₂O ; l'acétate de bismuth Bi(C₂H₃O₂)₃ ; le salicylate de bismuthyle C₆H₄CO₂(BiO)(OH).

La quantité d'activateur utilisée, exprimée par la quantité de métal contenue dans l'activateur par rapport au poids du métal M₁ engagé, peut varier dans de larges limites. Par exemple, cette quantité peut être aussi petite que 0,1 % et peut atteindre le poids de métal M₁ engagé et même le dépasser sans inconvénient.

Plus particulièrement, cette quantité est choisie de manière qu'elle apporte dans le milieu d'oxydation de 10 à 900 ppm en poids de métal activateur par rapport au mélange de composés phénoliques de formule (II). A cet égard, des quantités supérieures d'activateur de l'ordre de 900 à 1500 ppm peuvent naturellement être utilisées, mais sans avantage supplémentaire important.

Selon le procédé de l'invention, l'oxydation est conduite dans un milieu aqueux contenant en solution un agent basique, et plus particulièrement l'hydroxyde d'ammonium, les bases alcalines ou alcalino-terreuses parmi lesquelles on peut citer des hydroxydes tels que l'hydroxyde de sodium, de potassium, de lithium et la baryte ; des alcanolates alcalins tels que le méthylate, l'éthylate, l'isopropylate et le tert-butylate de sodium ou de potassium, des carbonates ou bicarbonates de sodium ou de potassium et de façon générale, les sels des bases alcalines ou alcalino-terreuses et d'acides faibles.

Ainsi, les composés de formule (III) à (V) peuvent être totalement ou partiellement salifiés selon la quantité d'agent basique mise en oeuvre. Il s'ensuit que dans lesdites formules, M, reste de la base, symbolise généralement un atome d'hydrogène et/ou un cation métallique du groupe (la) ou (IIa) ou un cation ammonium.

Pour des considérations économiques, on fait appel à l'hydroxyde de sodium ou de potassium. La proportion de base minérale à utiliser peut être comprise entre 0,5 et 10 moles, de préférence, entre 1 et 4 moles, et encore plus préférentiellement entre 2 et 4 moles de base minérale par mole de composés phénoliques de formule (II).

La concentration pondérale du mélange de composés phénoliques de formule (II) dans la phase liquide est habituellement comprise entre 1 % et 60 %, de préférence entre 2 % et 30 %.

Pratiquement une manière d'exécuter le procédé consiste à mettre en contact avec de l'oxygène moléculaire ou un gaz en contenant, par exemple l'air, la solution renfermant le mélange de composés phénoliques de formule (II), l'agent basique, le catalyseur à base de métal M₁, éventuellement l'activateur, selon les proportions indiquées ci-dessus.

On peut opérer à pression atmosphérique, mais l'on préfère travailler sous pression entre 1 et 20 bar.

Le mélange est ensuite agité à la température désirée jusqu'à consommation d'une quantité d'oxygène correspondant à celle nécessaire pour transformer le groupe hydroxyméthyle ou formyle des composés (A) et (C) en groupe carboxylique et le groupe hydroxyméthyle si présent des composés (A) et (B) en groupe formyle.

La température réactionnelle à adopter varie selon la stabilité thermique des produits à préparer.

Conformément à l'invention, la température est choisie de préférence, dans une gamme de température allant de 30°C à 200°C, de préférence, entre 40°C et 160°C.

La température est à adapter par l'Homme du Métier en fonction des conditions réactionnelles (en particulier quantité de base, nature du métal M₁, pression et agitation). Il a été trouvé notamment que plus la température est basse, plus la quantité d'agent basique à mettre en oeuvre est choisie élevée.

On précisera, à titre d'exemples, les conditions préférées dans le cas des métaux préférés, platine et palladium. Pour le platine, la température étant choisie entre 60°C à 160 °C, la quantité de base à utiliser est avantageusement comprise entre 1 et 3 moles, par mole de composés phénoliques de formule (II). Dans le cas du palladium, la température peut être choisie entre 30°C et 200°C, de préférence, entre 30°C et 150°C et pour ce dernier intervalle, la quantité de base est préférentiellement de 2 à 4 moles par mole de composés phénoliques.

Ainsi, la quantité de base doit être suffisante pour oxyder le groupement Y₁ en position ortho en groupe carboxylique. Elle est déterminée par l'Homme du Métier, en fonction de la température et du métal choisi.

En fin de réaction qui dure de préférence, entre 30 minutes et 6 heures, on récupère un mélange comprenant un 3-carboxy-4-hydroxybenzaldéhyde répondant préférentiellement à la formule (III), un 4-hydroxybenzaldéhyde répondant préférentiellement à la formule (V) et un acide 2-hydroxybenzoïque répondant préférentiellement à la formule (IV) : lesdits composés pouvant être partiellement ou totalement salifiés.

Puis, après refroidissement s'il y a lieu, on sépare la masse catalytique du milieu réactionnel, par exemple par filtration.

Selon une deuxième étape du procédé de l'invention, en fin de réaction, on soumet le milieu réactionnel à une réaction de décarboxylation.

Pour ce faire, on acidifie le milieu résultant par addition d'un acide protonique d'origine minérale, de préférence l'acide chlorhydrique ou l'acide sulfurique ou d'un acide organique tel que par exemple, l'acide triflurométhanesulfonique ou l'acide méthanesulfonique jusqu'à obtention d'un pH inférieur ou égal à 3.

La concentration de l'acide est indifférente et l'on fait appel de préférence, aux formes commerciales.

On chauffe le milieu réactionnel à une température variant par exemple entre 120°C et 350°C et, de préférence, de 150°C et 220°C.

Le procédé est conduit, de préférence, sous la pression autogène des réactifs.

En fin de réaction, on refroidit le milieu réactionnel entre 20°C et 80°C.

On obtient un milieu bi-phasique constitué d'une phase organique comprenant d'une part, le 4-hydroxybenzaldéhyde répondant préférentiellement à la formule (VI) et le composé phénolique de départ de formule (I) et d'autre part une phase aqueuse saline.

On sépare les phases organique et aqueuse et l'on récupère le 4-hydroxybenzaldéhyde à partir de la phase organique, selon les techniques classiques de séparation, par exemple, extraction à l'aide d'un solvant approprié (par exemple méthylisobutylcétone ou éther isopropylique) ou distillation.

Conformément au procédé perfectionné de l'invention, on part d'un mélange de deux composés phénoliques, l'un portant un groupe formyle ou hydroxyméthyle en position 2 et 4 et les deux autres portant un groupe formyle ou hydroxyméthyle en position 2 ou 4.

Ainsi, les mélanges de départ répondent plus particulièrement aux formules données ci-après : 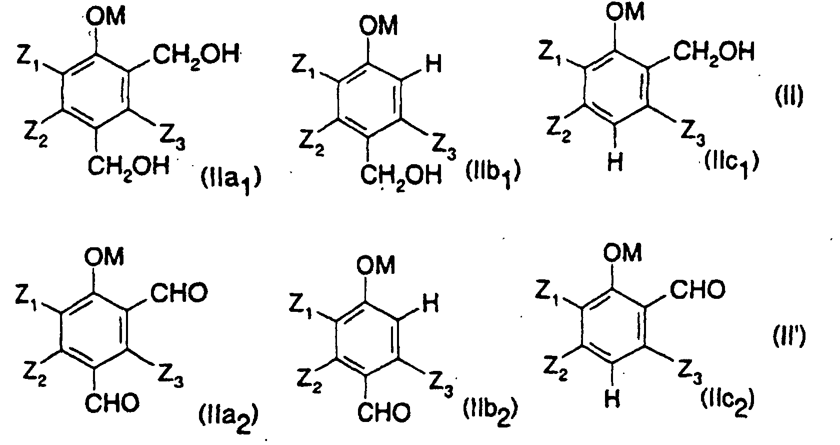 dans lesdites formules :
- M représente un atome d'hydrogène et/ou un cation métallique du groupe (la) ou (IIa) ou un cation ammonium,
- Z₁, Z₂, Z₃, ont les significations données précédemment.

Les mélanges de composés phénoliques, auxquels on peut appliquer le procédé selon l'invention sont préparés selon un procédé qui constitue un autre objet de l'invention.

Ainsi, des mélanges de composés phénoliques de formule (IIa₁) à (IIc₁) peuvent être obtenus par un procédé d'hydroxyméthylation d'un phénol par condensation de celui-ci avec du formaldéhyde ou d'un composé générateur de formaldéhyde en phase aqueuse en présence d'une base alcaline ou alcalino-terreuse, de telle sorte que le taux de conversion du phénol soit au plus de 95 %, suivie éventuellement d'une étape d'oxydation.

Plus précisément, on part d'un phénol non substitué sur les positions ortho et para par rapport au groupe hydroxyle, de formule générale (I) : 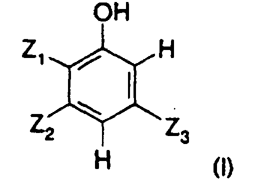 dans laquelle Z₁, Z₂, Z₃, ont les significations données précédemment.

Parmi les phénols de formule (I) qui peuvent servir de point de départ à la synthèse des composés de formule (II), on peut citer le gaïacol, le guétol, le 3-méthoxyphénol, le 3-éthoxyphénol, le 3-isopropoxyphénol, le 3-t-butoxyphénol, le m-crésol, l'o-crésol.

Les conditions choisies pour le déroulement de cette étape d'hydroxyméthylation sont celles enseignées par l'art antérieur rappelé ci-après : cf. notamment H.G. PEER Rec. Trav. Chim. Pays-Bas 79 825-835 (1960) ; GB-A-774 696 ; GB-A-751 845 ; EP-A-165 ; J.H. FREEMAN J. Am. Chem. Soc. 74 6 257-6 260 (1952) et 76 2080-2087 (1954) ; H.G. PEER Rec. Trav. Chim. Pays-Bas 78 851-863 (1959) ; H. EULER et al Arkiv für Chem. 13 1-7 (1939) ; P. CLAUS et al Monath. Chem. 103 1178-11293 (1972).

On peut faire appel au formaldéhyde ou tout générateur de formaldéhyde tel que, par exemple, le trioxane ou le paraformaldéhyde utilisé sous la forme de polyformaldéhydes linéaires de degré de polymérisation indifférent, ayant de préférence un nombre de motifs (CH₂O) compris entre 8 et 100 motifs,

Le formaldéhyde peut être utilisé, sous forme de solution aqueuse dont la concentration n'est pas critique. Elle peut varier entre 20 et 50 % en poids : on utilise de préférence les solutions commerciales dont la concentration est d'environ 30 à 40 % en poids.

La quantité de formaldéhyde exprimée en moles de formaldéhyde par mole de phénol peut varier dans de larges limites. Le rapport molaire formaldéhyde/phénol peut varier entre 1,0 et 4,0 et de préférence entre 1,0 et 2,5.

La quantité de base présente dans le milieu d'hydroxyméthylation exprimée par le nombre de moles de base/hydroxyle phénolique du phénol à hydroxyméthyler peut varier dans de larges limites. En général ce rapport, variable suivant la nature de la base, peut varier entre 1,0 et 4,0 et, de préférence, entre 0,9 et 2,0. Comme base, on peut utiliser celles citées plus haut pour la phase d'oxydation. L'emploi des hydroxydes alcalins en solution aqueuse est particulièrement commode.

En général, l'étape d'hydroxyméthylation est conduite à une température comprise entre 0 et 100°C et de préférence entre 20 et 70°C.

Le procédé est conduit, de préférence, sous la pression autogène des réactifs pour éviter les pertes éventuelles du paraformaldéhyde qui peut être gazeux aux températures de mises en oeuvre.

On préfère conduire la réaction sous atmosphère contrôlée de gaz inertes tels que l'azote ou les gaz rares, par exemple l'argon.

La durée de la réaction est déterminée aisément par l'Homme du Métier en fonction du taux de conversion du phénol de départ souhaité et en tenant compte de la nécessité de minimiser les sous-produits tels que les bis-arylméthane. Elle se situe, le plus souvent, entre 15 minutes et 4 heures, de préférence entre 1 heure et 3 heures.

Le taux de conversion du phénol sera contrôlé par différents paramètres (température, durée, quantité de réactifs). Il est avantageusement compris entre 60 et 95 %, de préférence, entre 80 et 95 %.

D'un point de vue pratique, la réaction est aisément réalisée en chargeant dans l'appareillage le phénol et le formaldéhyde, éventuellement une base, puis en portant le mélange réactionnel sous agitation à la température désirée, pendant la durée nécessaire à l'achèvement de la réaction.

L'ordre d'introduction des réactifs n'est pas critique et peut donc être différent.

On obtient un mélange de composés phénoliques de formule (IIa₁) à (IIc₁).

Les composés de formule (IIa₂) à (IIc₂) peuvent être préparés par oxydation des composés phénoliques hydroxyméthylés de formule (IIa₁) à (IIc₁), par oxydation par l'oxygène moléculaire ou un gaz qui en contient, en phase aqueuse alcaline en présence d'un catalyseur à base d'un métal du groupe 8 de la classification périodique, de préférence, le platine et le palladium, contenant éventuellement à titre d'activateur des métaux tels que le cadmium, le cérium, le bismuth, le plomb, l'argent, le tellure ou l'étain. De tels procédés ont été décrits dans US-A-3 673 257, FR-A-2 305 420 et dans FR-A-2 350 323.

Si cela est nécessaire, le pH de la solution est porté à une valeur comprise entre 8 et 13 par addition éventuelle d'une base alcaline ou alcalino-terreuse. La valeur optimale du pH dépend de la nature des phénols hydroxyméthylés.

La température de la réaction d'oxydation se situe entre 10°C et 100°C, et de préférence entre 20°C et 60°C.

Plus spécifiquement encore le procédé selon la présente invention convient bien à la préparation de composés de formule (IIa₂) à (IIc₂) à partir de composés phénoliques de formule (IIa₁) à (IIc₁) résultant de la première étape, par l'oxygène moléculaire ou un gaz qui en contient, en présence d'un catalyseur à base d'un métal du groupe 8 de la classification périodique, contenant éventuellement un métal tel que ceux utilisés à titre d'activateur, sans séparation intermédiaire des composés phénoliques hydroxyméthylés.

Il apparaît particulièrement avantageux du point de vue industriel pour la mise en oeuvre du procédé selon la présente invention de faire appel à des composés de formule (IIa₂) à (IIc₂) obtenus par un procédé en deux étapes comprenant :
- l'hydroxyméthylation d'un phénol en milieu aqueux en présence d'une base alcaline ou alcalino-terreuse par le formaldéhyde ou un composé générateur de formaldéhyde, conduisant à un mélange de composés phénoliques hydroxyméthylés, l'un hydroxyméthylé en position 2 et 4 et les deux autres hydroxyméthylés en position 2 ou en position 4,
- et l'oxydation, sans séparation intermédiaire, des composés phénoliques obtenus par l'oxygène moléculaire ou un gaz qui en contient, en phase aqueuse alcaline en présence d'un catalyseur à base d'un métal du groupe 8 de la classification périodique, éventuellement à titre d'activateur, un métal tel que ceux cités précédemment.

Un intérêt supplémentaire du procédé de l'invention est qu'il permet de mettre en oeuvre des mélanges de composés phénoliques issus directement des étapes précédentes d'hydroxyméthylation éventuellement d'oxydation.

Comme mentionné précédemment, le procédé de l'invention est particulièrement bien adapté pour la préparation de la vanilline et de l'éthylvanilline à partir d'un mélange de composés phénoliques obtenus par hydroxyméthylation du gaïacol ou du guétol.

Ainsi, la vanilline peut être préparée en soumettant un mélange de composés phénoliques, le 4,6-di(hydroxyméthyl)gaïacol (A), le p-hydroxyméthylgaïacol (B) et l'o-hydroxyméthylgaïacol (C) à une oxydation sélective du groupe hydroxyméthyle en position 2 des composés (A) et (C) en groupe carboxylique, et du groupe hydroxyméthyle des composés (A) et (B) en position 4 en groupe formyle, conduisant ainsi à un mélange de 3-carboxy-4-hydroxy-5-méthoxybenzaldéhyde, de vanilline et d'acide 2-hydroxy-3-méthoxybenzoique qui, après l'opération de décarboxylation conduit à la vanilline et au gaïacol qui peut être recyclé.

Une autre variante consiste à soumettre un mélange de composés phénoliques, le 4,6-di(formyl)gaïacol (A), le p-formylgaïacol (B) et l'o-formylgaïacol (C) à une oxydation sélective du groupe formyle en position 2 des composés (A) et (C) en groupe carboxylique, conduisant ainsi à un mélange de 3-carboxy-4-hydroxy-5-méthoxybenzaldéhyde, de vanilline et d'acide 2-hydroxy-3-méthoxybenzoïque qui, après l'opération de décarboxylation conduit à la vanilline et au gaïacol qui peut être recyclé.

En ce qui concerne la préparation d'éthylvanilline, selon l'invention, on soumet un mélange de composés phénoliques, le 4,6-di(hydroxyméthyl)guétol (A), le p-hydroxyméthylguétol (B) et l'o-hydroxyméthylguétol (C) à une oxydation sélective du groupe hydroxyméthyle en position 2 des composés (A) et (C) en groupe carboxylique, et du groupe hydroxyméthyle des composés (A) et (B) en position 4 en groupe formyle, conduisant ainsi à un mélange de 3-carboxy-4-hydroxy-5-éthoxybenzaldéhyde, d'éthylvanilline et d'acide 2-hydroxy-3-éthoxybenzoïque qui, après l'opération de décarboxylation conduit à l'éthylvanilline et au guétol qui peut être recyclé.

Une autre variante réside dans le fait que l'on part d'un mélange de composés phénoliques, le 4,6-di(formyl)guétol (A), le p-formylguétol (B) et l'o-formylguétol (C) à une oxydation sélective du groupe formyle en position 2 des composés (A) et (C) en groupe carboxylique, conduisant ainsi à un mélange de 3-carboxy-4-hydroxy-5-éthoxybenzaldéhyde, d'éthylvanilline et d'acide 2-hydroxy-3-éthoxybenzoïque qui, après l'opération de décarboxylation conduit à l'éthylvanilline et au guétol qui peut être recyclé.

On donne ci-après des exemples de réalisation de l'invention. Ces exemples sont donnés à titre illustratif et sans caractère limitatif.

Dans les exemples, on définit le taux de conversion et le rendement obtenu.

Le taux de conversion (TT) correspond au rapport entre le nombre de substrat transformées et le nombre de moles de substrat engagées.

Le rendement (RR) correspond au rapport entre le nombre de moles de produit formées et le nombre de moles de substrat engagées.

Le rendement (RTᵥₐₙᵢₗₗᵢₙₑ) correspond au rapport entre le nombre de moles de vanilline formées et le nombre de moles de gaïacol transformées sur l'enchaînement.

Dans les exemples, les abréviations signifient :
- o-hydroxyméthylgaiacol = OMG
- p-hydroxyméthylgaiacol = PMG
- o-vanilline = 3-méthoxy-2-hydroxybenzaldéhyde = OVA
- p-vanilline = 3-méthoxy-4-hydroxybenzaldéhyde = PVA
- acide o-vanillique = acide 2-hydroxy-3-méthoxybenzoïque = AOV
- acide p-vanillique = acide 4-hydroxy-3-méthoxybenzoïque = APV
- 4,6-di(hydroxyméthyl)gaïacol = DMG
- 4,6-(diformyl)gaïacol = DFG
- o-carboxyvanilline = OCVA
- 4,6-(dicarboxy)gaïacol = DCG

### Exemple 1

### 1 - Etape de condensation

Dans un réacteur de 2 litres muni d'une agitation mécanique et d'un dispositif de régulation de température, on charge :
- 152 g de gaïacol,
- 246 g d'une solution aqueuse de formol à 30 %,
- 49,2 g de soude
- et 672 g d'eau.

Le milieu réactionnel est maintenu 1 h à 45°C puis refroidi et dosé par chromatographie liquide haute performance.

Le bilan réactionnel est le suivant :
- conversion gaïacol = 90 %
- rendement o-hydroxyméthylgaïacol (OMG) = 15 %
   p-hydroxyméthylgaïacol (PMG) = 18 %
   4,6-di(hydroxyméthyl)gaiacol (DMG) = 50 %
- rendements (OMG + DMG + PMG) = 83 %
La somme des rendements des produits valorisables est de 93 %.

### 2 - Etape d'oxydation

Le milieu réactionnel est alors dilué par 1500 g d'eau et 148 g de soude.

Le milieu réactionnel est alors introduit dans un autoclave de 3,9 litres muni d'une turbine autoaspirante.

On ajoute alors à ce milieu réactionnel, 0,54 g de trioxide de bismuth et 22 g de catalyseur au palladium déposé sur noir à raison de 3 % en poids de métal.

On met sous agitation à 1500 t/min, et on monte la température du milieu réactionnel à 45°C sous azote. On se place alors sous pression de 3 bar et l'on introduit dans le milieu réactionnel de l'air avec un débit de 300 g/h. On maintient le milieu réactionnel 6 h dans ces conditions.

On refroidit alors le milieu réactionnel et l'on ramène la pression à la pression atmosphérique, puis l'on filtre le catalyseur.

Le milieu réactionnel est alors dosé par chromatographie liquide haute performance.

Les rendements sont les suivants (pour l'enchaînement complet) :
- TT gaïacol = 92 %
- série ortho
- RR o-hydroxyméthylgaïacol (OMG) = 0 %
- RR orthovanilline(OVA) = 1 %
- RR acide orthovanillique (AOV) = 14 %
- série para
- RR p-hydroxyméthylgaïacol (PMG) = 0 %
- RR vanilline (PVA) = 16 %
- RR acide paravanillique (APV) = 1 %
- série di
- RR 4,6-di(hydroxyméthyl)gaïacol (DMG) = 0 %
- RR 4,6-(diformyl)gaïacol (DFG) = 1 %
- RR orthocarboxyvanilline (OCVA) = 47 %
- RR 4,6-(dicarboxy)gaïacol (DCG) = 10 %

La somme des rendements des produits "valorisables" (gaïacol + OAV + PVA + APV + OCVA + DCG) est de 87 %.

### 3 - Décarboxylation du mélange réactionnel

199,91 g de ce mélange réactionnel sont neutralisés par 16,69 g d'acide sulfurique à 92 % et introduits dans un autoclave de 300 ml muni d'une turbine et d'un système de régulation de température.

Le milieu réactionnel est porté 3 h à 175°C sous pression autogène puis refroidi, et dosé en chromatographie liquide.

Le rendement en vanilline et la conversion en gaïacol sont les suivants :
- TT gaïacol = 76 % / gaïacol initial
- RR vanilline = 61 % / gaïacol initial soit RT vanilline = 80 %

### Exemple 2

### 1 - Etape de condensation

Dans un réacteur de 2 litres muni d'une agitation mécanique et d'un dispositif de régulation de température, on charge :
- 133 g de gaïacol,
- 202 g d'une solution aqueuse de formol à 30 % en poids,
- 145 g d'une solution aqueuse de soude à 30 % en poids,
- et 460 g d'eau.

Le milieu réactionnel est maintenu 0 h 50 à 47°C puis refroidi.

On ajoute 290 g d'une solution aqueuse de soude à 30 % en poids.

On dose par chromatographie liquide haute performance.

Le bilan réactionnel est le suivant :
- conversion gaïacol = 97 %
- rendement o-hydroxyméthylgaïacol (OMG) = 10 %
   p-hydroxyméthylgaïacol (PMG) = 12 %
   4,6-di(hydroxyméthyl)gaïacol (DMG) = 70 %
- rendements (OMG + DMG + PMG) = 92 %

La somme des rendements RT des produits valorisables est de 95 %.

### 2 - Etape d'oxydation

Le milieu réactionnel est alors dilué par 1230 g d'eau.

Le milieu réactionnel est alors introduit dans un autoclave de 3,9 litres muni d'une turbine autoaspirante.

On ajoute alors à ce milieu réactionnel, 0,54 g de trioxide de bismuth et 34,5 g de catalyseur au platine déposé sur noir à raison de 2 % en poids de métal.

On met sous agitation à 950 t/min, et on monte la température du milieu réactionnel à 70°C sous azote. On se place alors sous pression de 4 bar et l'on introduit dans le milieu réactionnel de l'air avec un débit de 200 g/h. On maintient le milieu réactionnel 5 h dans ces conditions.

On refroidit alors le milieu réactionnel et l'on ramène la pression à la pression atmosphérique, puis l'on filtre le catalyseur.

Le milieu réactionnel est alors dosé par chromatographie liquide haute performance.

Les rendements sont les suivants (pour l'enchaînement complet) :
- TT gaïacol = 97 %
- série ortho
- RR o-hydroxyméthylgaïacol (OMG) = 0 %
- RR orthovanilline(OVA) = 1 %
- RR acide orthovanillique (AOV) = 6 %
- série para
- RR p-hydroxyméthylgaiacol (PMG) = 0 %
- RR vanilline (PVA) = 9 %
- RR acide paravanillique (APV) = 2 %
- série di
- RR 4,6-di(hydroxyméthyl)gaïacol (DMG) = 0 %
- RR 4,6-(diformyl)gaïacol (DFG) = 1 %
- RR orthocarboxyvanilline (OCVA) = 54 %
- RR 4,6-(dicarboxy)gaïacol (DCG) = 6 %

La somme des rendements des produits "valorisables" (gaïacol + OAV + PVA + APV + OCVA + DCG) est de 80 %.

### 3 - Décarboxylation du mélange réactionnel

150 g de ce mélange réactionnel sont neutralisés par 15 ml d'acide sulfurique à 10 mol/l et introduits dans un autoclave de 300 ml muni d'une turbine et d'un système de régulation de température.

Le milieu réactionnel est porté 2 h à 175°C sous pression autogène puis refroidi, et dosé en chromatographie liquide.

Le rendement en vanilline et la conversion en gaïacol sont les suivants :
- TT gaïacol = 91 % / gaïacol initial
- RR vanilline = 52 % / gaïacol initial soit RT vanilline = 57 %

## Revendications

1. Procédé de préparation d'un 4-hydroxybenzaldéhyde et dérivés **caractérisé par** le fait qui consiste à oxyder sélectivement le groupe en position 2 par rapport au groupe hydroxyle, en groupe carboxylique et éventuellement le groupe hydroxyméthyle en position 4 en groupe formyle, présents dans les composés phénoliques d'un mélange comprenant au moins :
- un composé phénolique (A) porteur de groupes formyle et/ou hydroxyméthyle en position 2 et 4,
- un composé phénolique (B) porteur d'un groupe formyle ou hydroxyméthyle en position 4,
- un composé phénolique (C) porteur d'un groupe formyle ou hydroxyméthyle en position 2,
conduisant ainsi à un mélange comprenant un 3-carboxy-4-hydroxybenzaldéhyde, un 4-hydroxybenzaldéhyde, un acide 2-hydroxybenzoïque, qui est soumis ensuite à une opération de décarboxylation permettant ainsi d'obtenir le 4-hydroxybenzaldéhyde et un phénol qui peut être éventuellement recyclé.

2. Procédé de préparation d'un 4-hydroxybenzaldéhyde et dérivés **caractérisé par le fait que** l'on soumet un mélange comprenant au moins :
- un composé phénolique (A) porteur de groupes formyle et/ou hydroxyméthyle en position 2 et 4,
- un composé phénolique (B) porteur d'un groupe formyle ou hydroxyméthyle en position 4,
- un composé phénolique (C) porteur d'un groupe formyle ou hydroxyméthyle en position 2,
à une oxydation sélective du groupe en position 2 en groupe carboxylique et éventuellement d'un groupe hydroxyalkyle en position 4 en groupe formyle, en présence d'une base et d'un catalyseur à base d'un métal M1 choisis parmi les métaux 1b et 8, conduisant à un mélange comprenant un 3-carboxy-4-hydroxybenzaldéhyde, un 4-hydroxybenzaldéhyde, un acide 2-hydroxybenzoïque, qui est ensuite soumis à une opération de décarboxylation par chauffage permettant ainsi d'obtenir le 4-hydroxybenzaldéhyde et un phénol qui peut être éventuellement recyclé.

3. Procédé selon l'une des revendications 1 et 2 **caractérisé par le fait que** le mélange de composés phénoliques mis en oeuvre répond à la formule générale (II): 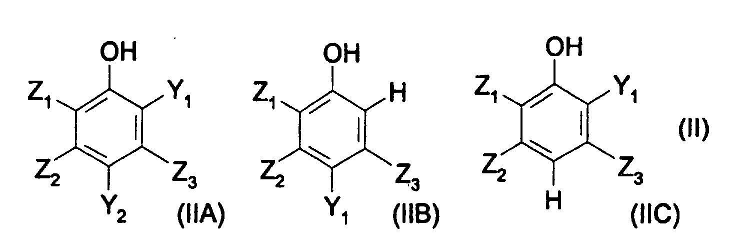 dans lesdites formules (IIA) à (IIC) :
- Y₁ et Y₂, identiques ou différents, représentent l'un des groupes suivants :
. un groupe - CHO,
. un groupe - CH₂OH,
- Z₁, Z₂ et Z₃, identiques ou différents, représentent un atome d'hydrogène, un radical alkyle, alcényle, alkoxy, hydroxyalkyle, alkoxyalkyle, cycloalkyle, aryle, un groupe hydroxyle, un groupe nitro, un atome d'halogène, un groupe trifluorométhyle.

4. Procédé selon l'une des revendications 1 à 3 **caractérisé par le fait que** les composés phénoliques répondent aux formules (IIA) à (IIC) dans lesquelles Z₁, Z₂ et Z₃, identiques ou différents, représentent l'un des atomes ou groupes suivants :
. un atome d'hydrogène,
. un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
. un radical alcényle, linéaire ou ramifié, ayant de 2 à 12 atomes de carbone, de préférence de 2 à 4 atomes de carbone, tel que vinyle, allyle,
. un radical alkoxy linéaire ou ramifié ayant de 1 à 12 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un radical phényle,
. un atome d'halogène, de préférence un atome de fluor, chlore ou brome.

5. Procédé selon l'une des revendications 1 à 4 **caractérisé par le fait que** les composés phénoliques répondent aux formules (IIA) à (IIC) dans lesquelles Z₁ représente un atome d'hydrogène ou un radical alkyle ou alkoxy, linéaire ou ramifié, ayant de 1 à 6 atomes de carbone, de préférence, 1 à 4 atomes de carbone ; Z₂ et Z₃ représentant un atome d'hydrogène ; Y₁ et Y₂, identiques, représentent un groupe formyle ou un groupe hydroxyméthyle.

6. Procédé selon l'une des revendications 1 à 5 **caractérisé par le fait que** le mélange de composés phénoliques répondant à la formule (II) est :
- l'o-hydroxyméthylgaïacol, le p-hydroxyméthylgaïacol et le 4,6-di(hydroxyméthyl)gaïacol,
- l'o-formylgaïacol, le p-formylgaïacol et le 4,6-diformylgaïacol,
- l'o-hydroxyméthylguétol, le p-hydroxyméthylguétol et le 4,6-di(hydroxyméthyl)guétol,
- l'o-formylguétol, le p-formylguétol et le 4,6-diformylguétol.

7. Procédé selon l'une des revendications 1 à 6 **caractérisé par le fait que** l'on oxyde le mélange de composés phénoliques de formule (II), en phase liquide, à l'aide d'oxygène moléculaire ou un gaz en contenant, en milieu aqueux renfermant un agent basique, en présence d'un catalyseur à base d'un métal M₁ choisi parmi les métaux du groupe 1b et 8 de la classification périodique des éléments, comprenant éventuellement, à titre d'activateurs des métaux tels que le cadmium, le cérium, le bismuth, le plomb, l'argent, le tellure ou l'étain.

8. Procédé selon la revendication 7 **caractérisé par le fait que** le catalyseur est à base de cuivre, de nickel, de ruthénium, de rhodium, de palladium, d'osmium, d'iridium, de platine ou de leurs mélanges : ledit catalyseur étant de préférence à base de platine et/ou de palladium.

9. Procédé selon l'une des revendications 7 et 8 **caractérisé par le fait que** le catalyseur au platine et/ou palladium, est apporté sous forme de noir de platine, de noir de palladium, d'oxyde de platine, d'oxyde de palladium ou de métal noble lui-même déposé sur des supports divers tels que le noir de carbone, le carbonate de calcium, les alumines et silices activées ou des matériaux équivalents, de préférence le noir de carbone.

10. Procédé selon l'une des revendications 7 à 9 **caractérisé par le fait que** la quantité de catalyseur à mettre en oeuvre, exprimée en poids de métal M₁ par rapport à celui du mélange de composés phénoliques de formule (II) peut varier de 0,01 à 10 % et, de préférence, de 0,04 à 2 %.

11. Procédé selon l'une des revendication 7 à 10 **caractérisé par le fait que** l'activateur est un dérivé organique ou inorganique du bismuth pris dans le groupe formé par : les oxydes de bismuth ; les hydroxydes de bismuth ; les sels de bismuth ou de bismuthyle d'hydracides minéraux, de préférence les chlorure, bromure, iodure, sulfure, sélénure, tellure ; les sels de bismuth ou de bismuthyle d'oxyacides minéraux, de préférence les sulfite, sulfate, nitrite, nitrate, phosphite, phosphate, pyrophosphate, carbonate, perchlorate, antimoniate, arséniate, sélénite, séléniate ; les sels de bismuth ou de bismuthyle d'acides organiques aliphatiques ou aromatiques, de préférence les acétate, propionate, salicylate, benzoate, oxalate, tartrate, lactate, citrate ; les phénates de bismuth ou de bismuthyle, de préférence les gallate et pyrogallate.

12. Procédé selon la revendication 11 **caractérisé par le fait que** le dérivé du bismuth est pris dans le groupe formé par : les oxydes de bismuth Bi₂O₃ et Bi₂O₄ ; l'hydroxyde de bismuth Bi(OH)₃ ; le chlorure de bismuth BiCl₃ ; le bromure de bismuth BiBr₃ ; l'iodure de bismuth Bil₃ ; le sulfate neutre de bismuth Bi₂(SO₄)₃ ; le nitrate neutre de bismuth Bi(NO₃)₃, 5H₂O ; le nitrate de bismuthyle BiO(NO₃) ; le carbonate de bismuthyle (BiO)₂CO₃, 0,5 H₂O ; l'acétate de bismuth Bi(C₂H₃O₂)₃ ; le salicylate de bismuthyle C₆H₄CO₂(BiO)OH.

13. Procédé selon l'une.des revendications 7 à 12 **caractérisé par le fait que** la quantité d'activateur est choisie de manière qu'elle apporte dans la milieu : d'une part, au moins 0,1 % en poids de métal activateur par rapport au poids du métal M₁ engagé, et d'autre part de 10 à 900 ppm en poids de métal M₁ par rapport au mélange de composés phénoliques de formule (II).

14. Procédé selon l'une des revendications 7 à 13 **caractérisé par le fait que** la réaction d'oxydation est conduite dans une gamme de température allant de 30°C à 200°C, de préférence, entre 40°C et 160°C.

15. Procédé selon l'une des revendications 7 à 14 **caractérisé par le fait que** l'on opère sous pression entre 1 et 20 bar.

16. Procédé selon l'une des revendications 7 à 15 **caractérisé par le fait que** l'oxydation est conduite dans un milieu aqueux contenant en solution un agent basique, de préférence, un hydroxyde de sodium, de potassium, en une quantité telle qu'elle représente de 0,5 à 10 moles, de préférence de 1 à 4 moles, et encore plus préférentiellement de 2 à 4 moles de base minérale par mole de composés phénoliques de formule (II).

17. Procédé selon la revendication 14 **caractérisé par le fait que** dans le cas d'un catalyseur au platine, la température est choisie entre 60°C et 160 °C ; la quantité de base à utiliser étant comprise entre 1 et 3 moles, par mole de composés phénoliques de formule (II).

18. Procédé selon la revendication 14 **caractérisé par le fait que** dans le cas d'un catalyseur au palladium, la température est choisie entre 30°C et 200°C, de préférence, entre 30°C et 150°C ; la quantité de base à utiliser étant comprise entre 2 et 4 moles, par mole de composés phénoliques de formule (II).

19. Procédé selon la revendication 1 à 18 **caractérisé par le fait que** l'on soumet le milieu réactionnel obtenu comprenant un 3-carboxy-4-hydroxybenzaldéhyde, un acide 2-hydroxybenzoïque, un 4-hydroxybenzaldéhyde totalement ou partiellement salifiés, à une opération de décarboxylation.

20. Procédé selon la revendication 19 **caractérisé par le fait que** les acides à décarboxyler répondent aux formules : 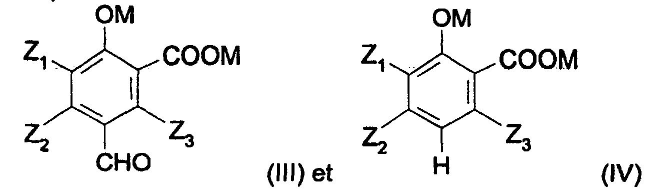 dans lesdites formules (III) et (IV) :
- M représente un atome d'hydrogène et/ou un cation métallique du groupe (la) ou (IIa) ou un cation ammonium,
- Z₁,Z₂,Z₃, ont les significations données dans les revendications 3 à 5.

21. Procédé selon la revendication 20 **caractérisé par le fait que** l'on effectue la décarboxylation dudit acide par addition dans le milieu réactionnel, d'un acide protonique d'origine minérale, de préférence l'acide chlorhydrique ou l'acide sulfurique ou organique, jusqu'à obtention d'un pH inférieur ou égal à 3.

22. Procédé selon l'une des revendications 20 à 22 **caractérisé par le fait que** l'on chauffe le milieu réactionnel à une température variant entre 120°C et 350°C et, de préférence, entre 150°C et 220°C et après refroidissement que l'on sépare le 4-hydroxybenzaldéhyde, et répondant préférentiellement à la formule (VI) : 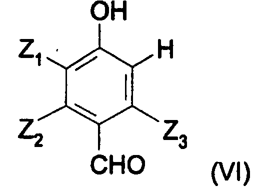 dans ladite formule (VI) :
- Z₁, Z₂, Z₃, ont les significations données dans les revendications 3 à 5.

23. Mélange réactionnel obtenu comprenant un 3-carboxy-4-hydroxybenzaldéhyde, un acide 2-hydroxybenzoïque, un 4-hydroxybenzaldéhyde totalement ou partiellement salifiés.

24. Procédé de préparation du mélange décrit dans la revendication 23 **caractérisé par le fait que** qu'il consiste à oxyder sélectivement selon le procédé décrit dans l'une des revendications 1 à 17, le groupe en position 2 par rapport au groupe hydroxyle, en groupe carboxylique, et éventuellement le groupe hydroxyméthyle en position 4 en groupe formyle, présents dans les composés phénoliques d'un mélange comprenant au moins :
- un composé phénolique (A) porteur de groupes formyle et/ou hydroxyméthyle en position 2 et 4,
- un composé phénolique (B) porteur d'un groupe formyle ou hydroxyméthyle en position 4,
- un composé phénolique (C) porteur d'un groupe formyle ou hydroxyméthyle en position 2.

25. Mélange de composés phénoliques totalement ou partiellement salifiés comprenant :
- un composé phénolique (A) porteur de groupes formyle et/ou hydroxyméthyle en position 2 et 4,
- un composé phénolique (B) porteur d'un groupe formyle ou hydroxyméthyle en position 4,
- un composé phénolique (C) porteur d'un groupe formyle ou hydroxyméthyle en position 2.

26. Mélange de composés phénoliques mis en oeuvre répondant à la formule générale (II) : 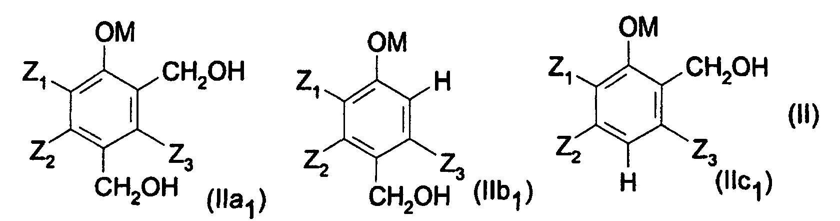 dans lesdites formules :
- M représente un atome d'hydrogène et/ou un cation métallique du groupe (la) ou (IIa) ou un cation ammonium,
- Z₁, Z₂, Z₃, ont les significations données dans les revendications 3 à 5.

27. Mélange de composés phénoliques mis en oeuvre répondant à la formule générale (II') : 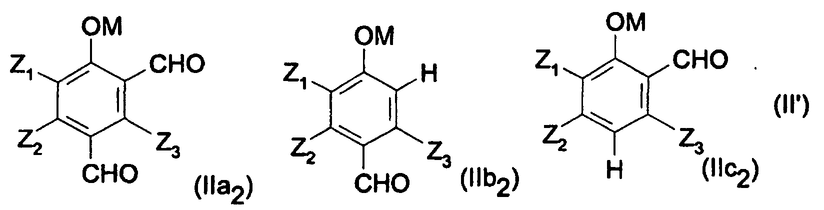 dans lesdites formules :
- M représente un atome d'hydrogène et/ou un cation métallique du groupe (la) ou (IIa) ou un cation ammonium,
- Z₁, Z₂, Z₃, ont les significations données dans les revendications 3 à 5.

28. Mélange de composés phénoliques selon l'une des revendications 24 à 26 **caractérisé par le fait qu'**il comprend :
- de 30 à 70 % en poids, et plus préférentiellement de 50 à 70 % d'un composé phénolique (A),
- de 30 à 70 % en poids, et plus préférentiellement de 50 à 70 % d'un mélange de composés phénoliques (B + C).

29. Procédé de préparation d'un mélange de composés phénoliques décrit dans l'une des revendications 25 à 28 **caractérisé par le fait qu'**il est obtenu par un procédé d'hydroxyméthylation d'un phénol par condensation de celui-ci avec du formaldéhyde ou d'un composé générateur de formaldéhyde en phase aqueuse en présence d'une base alcaline ou alcalino-terreuse de telle sorte que le taux de conversion du phénol soit au plus de 95 %, suivie éventuellement d'une étape d'oxydation.

30. Procédé selon la revendication 29 **caractérisé par le fait que** le taux de conversion du phénol varie de 60 à 95 %, de préférence, entre 80 et 95 %.

31. Procédé selon l'une des revendications 29 et 30 **caractérisé par le fait que** le phénol de départ est un phénol non substitué sur les positions ortho et para par rapport au groupe hydroxyle, de formule générale (I) : 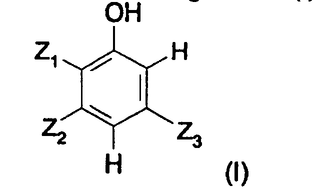 dans laquelle Z₁, Z₂, Z₃, ont les significations données précédemment.

32. Procédé selon la revendication 31 **caractérisé par le fait que** le phénol de formule (I) est le gaïacol, le guétol, le 3-méthoxyphénol, le 3-éthoxyphénol, le 3-isopropoxyphénol, le 3-t-butoxyphénol, le m-crésol, l'o-crésol.

33. Procédé selon l'une des revendications 29 à 32 **caractérisé par le fait que** l'on met en oeuvre le formaldéhyde ou tout générateur de formaldéhyde de préférence, le trioxane ou le paraformaldéhyde utilisé sous la forme de polyformaldéhydes linéaires de degré de polymérisation indifférent, ayant de préférence un nombre de motifs (CH₂O) compris entre 8 et 100 motifs.

34. Procédé selon la revendication 33 **caractérisé par le fait que** le rapport molaire formaldéhyde/phénol varie entre 1,0 et 4,0 et de préférence entre 1,0 et 2,5.

35. Procédé selon la revendication 29 **caractérisé par le fait que** la quantité de base présente dans le milieu d'hydroxyméthylation exprimée par le nombre de moles de base/hydroxyle phénolique du phénol à hydroxyméthyler varie entre 1,0 et 4,0 et, de préférence, entre 0,9 et 2,0.

36. Procédé selon la revendication 29 **caractérisé par le fait que** la température d'hydroxyméthylation est comprise entre 0 et 100°C et de préférence entre 20 et 70°C.

37. Procédé selon la revendication 29 **caractérisé par le fait que** la durée de la réaction d'hydroxyméthylation se situe entre 15 minutes et 4 heures, de préférence entre 1 heure et 3 heures.

38. Procédé selon la revendication 29 **caractérisé par le fait que** l'on charge dans l'appareillage le phénol et le formaldéhyde, éventuellement une base, puis l'on porte le mélange réactionnel sous agitation à la température désirée, pendant la durée nécessaire à l'achèvement de la réaction jusqu'à obtention d'un mélange de composés phénoliques de formule (IIa₁) à (IIc₁).

39. Procédé selon la revendication 27 **caractérisé par le fait qu'**un mélange de composés de formule (IIa₂) à (IIc₂) est obtenu par oxydation de des composés phénoliques hydroxyméthylés de formule (IIa₁) à (IIc₁), par oxydation par l'oxygène moléculaire ou un gaz qui en contient, en phase aqueuse alcaline en présence d'un catalyseur à base d'un métal du groupe 8 de la classification périodique, de préférence, le platine et le palladium, contenant éventuellement à titre d'activateur des métaux tels que le cadmium, le cérium, le bismuth, le plomb, l'argent, le tellure ou l'étain.

40. Procédé selon la revendication 39 **caractérisé par le fait que** le pH de la solution est porté à une valeur comprise entre 8 et 13 par addition éventuelle d'une base alcaline ou alcalino-terreuse.

41. Procédé selon la revendication 39 **caractérisé par le fait que** la température de la réaction d'oxydation se situe entre 10°C et 100°C, et de préférence entre 20°C et 60°C.

42. Procédé selon la revendication 27 **caractérisé par le fait qu'**un mélange de composés phénoliques de formule (IIa₂) à (IIc₂) est obtenu par un procédé en deux étapes comprenant :
- l'hydroxyméthylation d'un phénol en milieu aqueux en présence d'une base alcaline ou alcalino-terreuse par le formaldéhyde ou un composé générateur de formaldéhyde, conduisant à un mélange de composés phénoliques hydroxyméthylés, l'un hydroxyméthylé en position 2 et 4 et les deux autres hydroxyméthylés en position 2 ou en position 4,
- et l'oxydation, sans séparation intermédiaire, des composés phénoliques obtenus par l'oxygène moléculaire ou un gaz qui en contient, en phase aqueuse alcaline en présence d'un catalyseur à base d'un métal du groupe 8 de la classification périodique, éventuellement à titre d'activateur, un métal tel que ceux cités précédemment.

43. Procédé de préparation de la vanilline selon l'une des revendications 1 à 41 **caractérisé par le fait que** l'on soumet un mélange de composés phénoliques, le 4,6-di(hydroxyméthyl)gaïacol (A), le p-hydroxyméthylgaïacol (B) et l'o-hydroxyméthylgaïacol (C) à une oxydation sélective du groupe hydroxyméthyle en position 2 des composés (A) et (C) en groupe carboxylique, et du groupe hydroxyméthyle des composés (A) et (B) en position 4 en groupe formyle, conduisant ainsi à un mélange de 3-carboxy-4-hydroxy-5-méthoxybenzaldéhyde, de vanilline et d'acide 2-hydroxy-3-méthoxybenzoïque qui, après l'opération de décarboxylation conduit à la vanilline et au gaïacol qui peut être recyclé.

44. Procédé de préparation de vanilline selon l'une des revendications 1 à 42 **caractérisé par le fait que** l'on soumet un mélange de composés phénoliques, le 4,6-di(formyl)gaïacol (A), le p-formylgaïacol (B) et l'o-formylgaïacol (C) à une oxydation sélective du groupe formyle en position 2 des composés (A) et (C) en groupe carboxylique, conduisant ainsi à un mélange de 3-carboxy-4-hydroxy-5-méthoxybenzaldéhyde, de vanilline et d'acide 2-hydroxy-3-méthoxybenzoïque qui, après l'opération de décarboxylation conduit à la vanilline et au gaïacol qui peut être recyclé.

45. Procédé de préparation d'éthylvanilline selon l'une des revendications 1 à 41 **caractérisé par le fait que** l'on soumet un mélange de composés phénoliques, le 4,6-di(hydroxyméthyl)guétol (A), le p-hydroxyméthylguétol (B) et l'o-hydroxyméthylguétol (C) à une oxydation sélective du groupe hydroxyméthyle en position 2 des composés (A) et (C) en groupe carboxylique, et du groupe hydroxyméthyle des composés (A) et (B) en position 4 en groupe formyle, conduisant ainsi à un mélange de 3-carboxy-4-hydroxy-5-éthoxybenzaldéhyde, d'éthylvanilline et d'acide 2-hydroxy-3-éthoxybenzoïque qui, après l'opération de décarboxylation conduit à l'éthylvanilline et au guétol qui peut être recyclé.

46. Procédé de préparation d'éthylvanilline selon l'une des revendications 1 à 45 **caractérisé par le fait que** l'on soumet un mélange de composés phénoliques, le 4,6-di(formyl)guétol (A), le p-formylguétol (B) et l'o-formylguétol (C) à une oxydation sélective du groupe formyle en position 2 des composés (A) et (C) en groupe carboxylique, conduisant ainsi à un mélange de 3-carboxy-4-hydroxy-5-éthoxybenzaldéhyde, d'éthylvanilline et d'acide 2-hydroxy-3-éthoxybenzoïque qui, après l'opération de décarboxylation conduit à l'éthylvanilline et au guétol qui peut être recyclé.

47. Utilisation du procédé décrit dans l'une des revendications 1 à 46 pour la préparation de la vanilline et de l'éthylvanilline.

## Patentansprüche

1. Verfahren zur Herstellung eines 4-Hydroxybenzaldehyds und seiner Derivate, **dadurch gekennzeichnet, daß** man selektiv, jeweils bezogen auf die Hydroxylgruppe, die Gruppe in 2-Position zu einer Carboxylgruppe und gegebenenfalls die Hydroxymethylgruppe in 4-Position zu einer Formylgruppe oxidiert, die in den Phenolverbindungen einer Mischung vorhanden sind, welche mindestens umfaßt:
- eine Phenolverbindung (A), die Träger von Formyl- und/oder Hydroxymethylgruppen in 2- und 4-Position ist,
- eine Phenolverbindung (B), die Träger einer Formyl- oder Hydroxymethylgruppe in 4-Position ist,
- einer Phenolverbindung (C), die Träger einer Formyl- oder Hydroxymethylgruppe in 2-Position ist,
wobei das Verfahren zu einer Mischung aus 3-Carboxy-4-hydroxybenzaldehyd, 4-Hydroxybenzaldehyd und 2-Hydroxybenzoesäure führt, wobei die Mischung anschließend einer Decarboxylierung unterzogen wird, um 4-Hydroxybenzaldehyd und ein Phenol, das gegebenenfalls rezykliert werden kann, zu erhalten.

2. Verfahren zur Herstellung eines 4-Hydroxybenzaldehyds und seiner Derivate, **dadurch gekennzeichnet, daß** man eine Mischung aus mindestens
- einer Phenolverbindung (A), die Träger von Formyl- und/oder Hydroxymethylgruppen in 2- und 4-Position ist,
- einer Phenolverbindung (B), die Träger einer Formyl- oder Hydroxymethylgruppe in 4-Position ist,
- einer Phenolverbindung (C), die Träger einer Formyl- oder Hydroxymethylgruppe in 2-Position ist,
selektiv einer Oxidation der Gruppe in 2-Position zu einer Carboxylgruppe und gegebenenfalls einer Hydroxyalkylgruppe in 4-Position zu einer Formylgruppe in Gegenwart einer Base und eines Katalysators auf Basis eines Metalls M1 unterzieht, das ausgewählt ist aus den Metallen der Gruppen 1b und 8 des Periodensystems der Elemente, wobei die Oxidation zu einer Mischung aus 3-Carboxy-4-hydroxybenzaldehyd, 4-Hydroxybenzaldehyd und 2-Hydroxybenzoesäure führt, wobei die Mischung anschließend einer Decarboxylierung durch Erwärmung unterzogen wird, um ein 4-Hydroxybenzaldehyd und ein Phenol, das gegebenenfalls rezykliert werden kann, zu erhalten.

3. Verfahren nach einem der Ansprüche 1 oder 2, **dadurch gekennzeichnet, daß** die verwendete Mischung der Phenolverbindungen der allgemeinen Formel (II) entspricht: 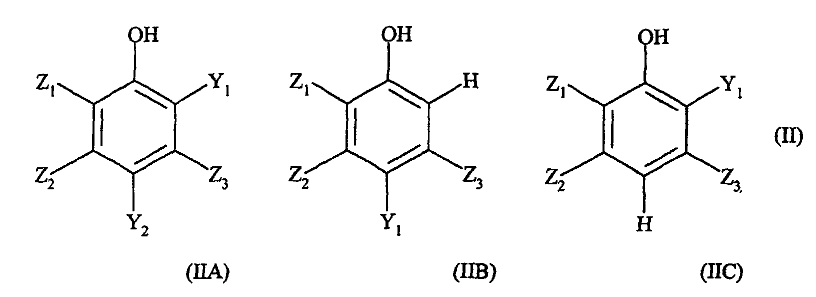 wobei in den Formeln (IIA) bis (IIC):
- Y₁ und Y₂, identisch oder verschieden, eine der folgenden Gruppen darstellen:
- eine -CHO-Gruppe,
- eine -CH₂OH-Gruppe,
- Z₁, Z₂ und Z₃, identisch oder verschieden, ein Wasserstoffatom, einen Alkyl-, Alkenyl-, Alkoxy-, Hydroxyalkyl-, Alkoxyalkyl-, Cycloalkyl-, Arylrest, eine Hydroxylgruppe, eine Nitrogruppe, ein Halogenatom oder eine Trifluormethylgruppe darstellen.

4. Verfahren nach einem der Ansprüche 1 bis 3, **dadurch gekennzeichnet, daß** die Phenolverbindungen den Formeln (IIA) bis (IIC) entsprechen, in denen Z₁, Z₂ und Z₃, identisch oder verschieden, einem der folgenden Atome oder einer der folgenden Gruppen entsprechen:
- einem Wasserstoffatom,
- einem linearen oder verzweigten Alkylrest mit 1 bis 12 Kohlenstoffatomen, vorzugsweise 1 bis 4 Kohlenstoffatomen, wie Methyl, Ethyl, Propyl, Isopropyl, Butyl, Isobutyl, sec.-Butyl, tert.-Butyl,
- einem linearen oder verzweigten Alkenylrest mit 2 bis 12 Kohlenstoffatomen, vorzugsweise 2 bis 4 Kohlenstoffatomen, wie Vinyl, Allyl,
- einem linearen oder verzweigten Alkoxyrest mit 1 bis 12 Kohlenstoffatomen, vorzugsweise 1 bis 4 Kohlenstoffatomen, wie Methoxy-, Ethoxy-, Propoxy-, Isopropoxy-, Butoxy-, sec.-Butoxy, tert.-Butoxyreste,
- einem Phenylrest,
- einem Halogenatom, vorzugsweise einem Fluor-, Chlor- oder Bromatom.

5. Verfahren nach einem der Ansprüche 1 bis 4, **dadurch gekennzeichnet, daß** die Phenolverbindungen den Formeln (IIA) bis (IIC) entsprechen, in denen Z₁ ein Wasserstoffatom oder einen linearen oder verzweigten Alkyl- oder Alkoxyrest mit 1 bis 6 Kohlenstoffatomen, vorzugsweise 1 bis 4 Kohlenstoffatomen, Z₂ und Z₃ ein Wasserstoffatom, und Y₁ und Y₂, identisch oder verschieden, eine Formyl- oder eine Hydroxymethylgruppe darstellen.

6. Verfahren nach einem der Ansprüche 1 bis 5, **dadurch gekennzeichnet, daß** die Mischung der Phenolverbindungen, die der Formel (II) entsprechen, ist:
- o-Hydroxymethylguajakol, p-Hydroxymethylguajakol und 4,6-Di(hydroxymethyl)guajakol,
- o-Formylguajakol, p-Formylguajakol und 4,6-Diformylguajakol,
- o-Hydroxymethylguetol, p-Hydroxymethylguetol und 4,6-(Dihydroxymethyl)guetol,
- o-Formylguetol, p-Formylguetol und 4,6-Diformylguetol.

7. Verfahren nach einem der Ansprüche 1 bis 6, **dadurch gekennzeichnet, daß** man die Mischung der Phenolverbindungen der Formel (II) in flüssiger Phase mit Hilfe von molekularem Sauerstoff oder einem sauerstoffhaltigen Gas in wäßriger Phase, die ein basisches Reagenz enthält, in Gegenwart eines Katalysators auf Basis eines Metalls M₁ oxidiert, das ausgewählt ist aus den Metallen der Gruppe 1b und 8 des Periodensystems der Elemente, wobei der Katalysator gegebenenfalls als Aktivator Metalle wie Cadmium, Cer, Bismut, Blei Silber, Tellur oder Zinn umfaßt.

8. Verfahren nach Anspruch 7, **dadurch gekennzeichnet, daß** der Katalysator ein Katalysator auf Basis von Kupfer, Nickel, Ruthenium, Rhodium, Palladium, Osmium, Iridium, Platin oder ihren Mischungen ist, wobei der Katalysator vorzugsweise auf Platin und/oder Palladium basiert.

9. Verfahren nach einem der Ansprüche 7 oder 8, **dadurch gekennzeichnet, daß** der Platin- und/oder Palladiumkatalysator bereitgestellt wird in Form von Platinschwarz, Palladiumschwarz, Platinoxid, Palladiumoxid oder als Edelmetall selbst, aufgebracht auf unterschiedlichen Trägern wie Ruß, Calciumcarbonat, aktivierten Aluminiumoxiden und Siliciumoxiden oder gleichwertigen Materialien, vorzugsweise Ruß.

10. Verfahren nach einem der Ansprüche 7 bis 9, **dadurch gekennzeichnet, daß** die Menge an zu verwendendem Katalysator, berechnet in Gew.-% des Metalls M₁ im Verhältnis zur Menge der Mischung der Phenolverbindungen der Formel (II), von 0,01 bis 10 %, vorzugsweise 0,04 bis 2 %, variieren kann.

11. Verfahren nach einem der Ansprüche 7 bis 10, **dadurch gekennzeichnet, daß** der Aktivator ein organisches oder anorganisches Derivat des Bismuts ist, ausgewählt aus der Gruppe der Bismutoxide; Bismuthydroxide; Bismutoder Bismuthylsalze der anorganischen Wasserstoffsäuren, vorzugsweise Chloride, Bromide, Iodide, Sulfide, Selenide, Telluride; Bismut- oder Bismuthylsalze der anorganischen Oxysäuren, vorzugsweise Sulfite, Sulfate, Nitrite, Nitrate, Phosphite, Phosphate, Pyrophosphate, Carbonate, Perchlorate, Antimonate, Arsenate, Selenite, Selenate; Bismut- oder Bismuthylsalze der aliphatischen oder aromatischen organischer Säuren, vorzugsweise Acetate, Propionate, Salicylate, Benzoate, Oxalate, Tartrate, Lactate, Citrate; Bismut- oder Bismuthylphenate, vorzugsweise Gallate oder Pyrogallate.

12. Verfahren nach Anspruch 11, **dadurch gekennzeichnet, daß** das Bismutderivat ausgewählt ist aus der Gruppe von Bismutoxiden Bi₂O₃ und Bi₂O₄, Bismuthydroxid Bi(OH)₃, Bismutchlorid BiCl₃, Bismutbromid BiBr₃, Bismutiodid BiI₃, neutralem Bismutnitrat Bi(NO₃)₃·5H₂O, Bismuthylnitrat BiO(NO₃), Bismuthylcarbonat (BiO)₂CO₃ · 0,5 H₂O, Bismutacetat Bi(C₂H₃O₂)₃, Bismuthylsalicylat C₆H₄CO₂(BiO)OH.

13. Verfahren nach einem der Ansprüche 7 bis 12, **dadurch gekennzeichnet, daß** die Menge des Aktivators so ausgewählt wird, daß sie einerseits mindestens 0,1 Gew.-% Aktivatormetall im Verhältnis zu verwendetem Metall M₁ und andererseits 10 bis 900 Gew.-ppm Metall M₁ im Verhältnis zur Mischung der Phenolverbindungen der Formel (II) in das Milieu einbringt.

14. Verfahren nach einem der Ansprüche 7 bis 13, **dadurch gekennzeichnet, daß** die Oxidationsreaktion in einem Temperaturbereich von 30 bis 200 °C, vorzugsweise von 40 bis 160 °C, durchgeführt wird.

15. Verfahren nach einem der Ansprüche 7 bis 14, **dadurch gekennzeichnet, daß** man unter einem Druck zwischen 1 und 20 bar arbeitet.

16. Verfahren nach einem der Ansprüche 7 bis 15, **dadurch gekennzeichnet, daß** die Oxidation in einem wäßrigen Milieu durchgeführt wird, das in Lösung ein basisches Reagenz, vorzugsweise Natrium- oder Kaliumhydroxid, in einer Menge enthält, die 0,5 bis 10 mol, insbesondere 1 bis 4 mol, vorzugsweise 2 bis 4 mol der anorganischen Base pro mol Phenolverbindungen der Formel (II), entspricht.

17. Verfahren nach Anspruch 14, **dadurch gekennzeichnet, daß** im Fall eines Platinkatalysators die Temperatur zwischen 60 °C und 160 °C ausgewählt wird und die Menge an zu verwendender Base zwischen 1 und 3 mol pro mol der Phenolverbindungen der Formel (II) beträgt.

18. Verfahren nach Anspruch 14, **dadurch gekennzeichnet, daß** im Fall eines Palladiumkatalysators die Temperatur zwischen 30 °C und 200 °C, vorzugsweise 30 °C und 150 °C, ausgewählt wird und die Menge an zu verwendender Base zwischen 2 und 4 mol pro mol Phenolverbindungen der Formel (II) beträgt.

19. Verfahren nach einem der Ansprüche 1 bis 18, **dadurch gekennzeichnet, daß** man das erhaltene Reaktionsmilieu aus 3-Carboxy-4-hydroxybenzaldehyd, 2-Hydroxybenzoesäure und 4-Hydroxybenzaldehyd, vollständig oder teilweise in Salzform, einer Decarboxylierung unterzieht.

20. Verfahren nach Anspruch 19, **dadurch gekennzeichnet, daß** die zu decarboxylierenden Säuren den Formeln entsprechen: 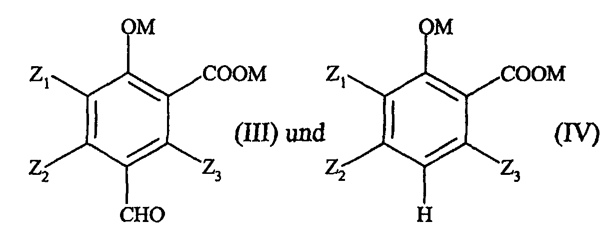 wobei in den Formeln (III) und (IV):
- M ein Wasserstoffatom und/oder ein Metallkation der Gruppe (Ia) oder (IIa) oder ein Ammoniumkation darstellt,
- Z₁, Z₂, Z₃ die Bedeutungen wie in den Ansprüchen 3 bis 5 haben.

21. Verfahren nach Anspruch 20, **dadurch gekennzeichnet, daß** man die Decarboxylierung der Säure durch Zugabe einer Protonensäure anorganischer Herkunft, vorzugsweise Salzsäure oder Schwefelsäure, oder organischer Herkunft bis zum Erhalt eines pH-Wertes kleiner oder gleich 3 durchführt.

22. Verfahren nach einem der Ansprüche 20 bis 22, **dadurch gekennzeichnet, daß** man das Reaktionsmilieu auf eine Temperatur zwischen 120 °C und 350 °C, vorzugsweise 150 °C und 220 °C, erwärmt und daß man nach Abkühlung den 4-Hydroxybenzaldehyd abtrennt, der vorzugsweise der Formel (VI) entspricht: 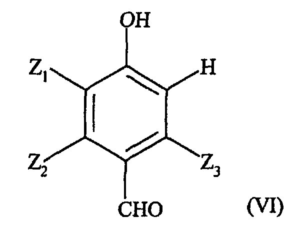 wobei in der Formel (VI):
- Z₁, Z₂, Z₃ die Bedeutungen wie in den Ansprüchen 3 bis 5 haben.

23. Erhaltene Reaktionsmischung, enthaltend 3-Carboxy-4-hydroxybenzaldehyd, 2-Hydroxybenzoesäure und 4-Hydroxybenzaldehyd, vollständig oder teilweise in Salzform.

24. Verfahren zur Herstellung einer in Anspruch 23 beschriebenen Mischung, **dadurch gekennzeichnet, daß** man gemäß dem in einem der Ansprüche 1 bis 17 beschriebenen Verfahren selektiv die bezüglich der Hydroxylgruppen in 2-Position befindliche Gruppe zu einer Carboxylgruppe und gegebenenfalls die Hydroxymethylgruppe in 4-Position zu einer Formylgruppe oxidiert, die in den Phenolverbindungen einer Mischung vorhanden sind, welche mindestens umfaßt:
- eine Phenolverbindung (A), die Träger von Formyl- und/oder Hydroxymethylgruppen in 2- und 4-Position ist,
- eine Phenolverbindung (B), die Träger einer Formyl- oder Hydroxymethylgruppe in 4-Position ist,
- einer Phenolverbindung (C), die Träger einer Formyl- oder Hydroxymethylgruppe in 2-Position ist.

25. Mischung von Phenolverbindungen, vollständig oder teilweise in Salzform, umfassend:
- eine Phenolverbindung (A), die Träger von Formyl- und/oder Hydroxymethylgruppen in 2- und 4-Position ist,
- eine Phenolverbindung (B), die Träger einer Formyl- oder Hydroxymethylgruppe in 4-Position ist,
- eine Phenolverbindung (C), die Träger einer Formyl- oder Hydroxymethylgruppe in 2-Position ist.

26. Mischungen von verwendeten Phenolverbindungen, die der allgemeinen Formel (II) entsprechen: 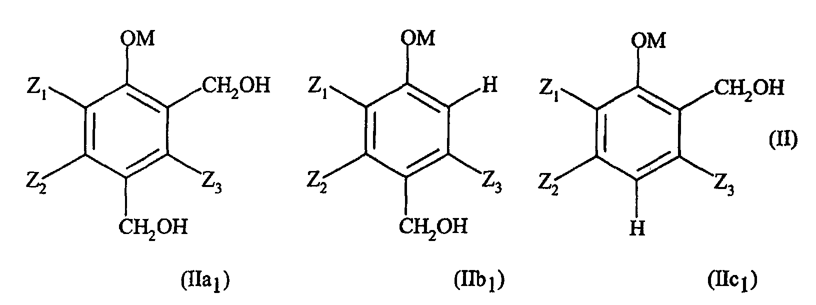 wobei in den Formeln:
- M ein Wasserstoffatom und/oder ein Metallkation der Gruppe (Ia) oder (IIa) oder ein Ammoniumkation darstellt,
- Z₁, Z₂, Z₃ die Bedeutungen wie in den Ansprüchen 3 bis 5 haben.

27. Mischungen von verwendeten Phenolverbindungen, die der allgemeinen Formel (II') entsprechen: 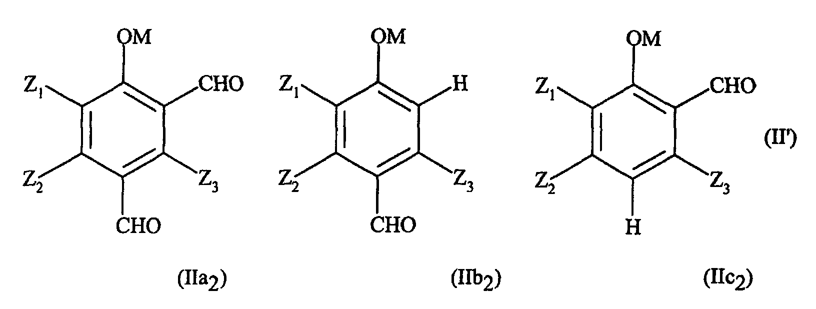 wobei in den Formeln:
- M ein Wasserstoffatom und/oder ein Metallkation der Gruppe (Ia) oder (IIa) oder ein Ammoniumkation darstellt,
- Z₁, Z₂, Z₃ die Bedeutungen wie in den Ansprüchen 3 bis 5 haben.

28. Mischungen von Phenolverbindungen gemäß einem der Ansprüche 24 bis 26, umfassend:
- 30 bis 70 Gew.-%, vorzugsweise 50 bis 70 Gew.-%, einer Phenolverbindung (A),
- 30 bis 70 Gew.-%, vorzugsweise 50 bis 70 Gew.-%, einer Mischung der Phenolverbindungen (B+C).

29. Verfahren zur Herstellung einer in einem der Ansprüche 25 bis 28 beschriebenen Mischung aus Phenolverbindungen, **dadurch gekennzeichnet, daß** sie durch Hydroxymethylierung eines Phenols durch dessen Kondensation mit Formaldehyd oder einem Formaldehyd-Erzeuger in wäßriger Phase in Gegenwart einer alkalischen oder erdalkalischen Base, so daß der Umsatz an Phenol größer als 95 % ist, und einen sich gegebenenfalls anschließenden Oxidationsschritt erhalten wird.

30. Verfahren nach Anspruch 29, **dadurch gekennzeichnet, daß** der Umsatz an Phenol von 60 bis 95 %, vorzugsweise zwischen 80 und 95%, variiert.

31. Verfahren nach einem der Ansprüche 29 oder 30, **dadurch gekennzeichnet, daß** das Ausgangsphenol ein in den ortho- und para-Positionen, bezogen auf die Hydroxylgruppe, nichtsubstituiertes Phenol der allgemeinen Formel (I) ist: 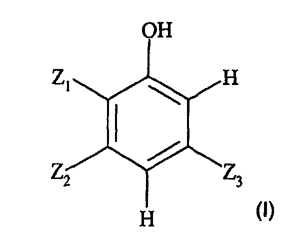 in der Z₁, Z₂, Z₃ den zuvor gegebenen Bedeutungen entspricht.

32. Verfahren nach Anspruch 31, **dadurch gekennzeichnet, daß** das Phenol der Formel (I) Guajakol, Guetol, 3-Methoxyphenol, 3-Ethoxyphenol, 3-Isopropoxyphenol, 3-tert.-Butylphenol, m-Kresol oder o-Kresol ist.

33. Verfahren nach einem der Ansprüche 29 bis 32, **dadurch gekennzeichnet, daß** man Formaldehyd oder jeden beliebigen Formaldehyd-Erzeuger, vorzugsweise Trioxan oder in Form von linearen Polyformaldehyden mit unterschiedlichem Polymerisationsgrad verwendetes Paraformaldehyd, mit einer Anzahl an (CH₂O)-Einheiten vorzugsweise zwischen 8 und 100 einsetzt.

34. Verfahren nach Anspruch 33, **dadurch gekennzeichnet, daß** das molare Verhältnis Formaldehyd/Phenol zwischen 1,0 und 4,0, vorzugsweise zwischen 1,0 und 2,5, variiert.

35. Verfahren nach Anspruch 29, **dadurch gekennzeichnet, daß** die in dem Milieu der Hydroxymethylierung vorhandene Menge an Base im Verhältnis zu dem zu hydroxymethylierenden Phenol, berechnet als Molzahl Base/Hydroxyphenol, zwischen 1,0 und 4,0, vorzugsweise 0,9 und 2,0, variiert.

36. Verfahren nach Anspruch 29, **dadurch gekennzeichnet, daß** die Temperatur der Hydroxymethylierung zwischen 0 und 100 °C, vorzugsweise zwischen 20 und 70 °C, variiert.

37. Verfahren nach Anspruch 29, **dadurch gekennzeichnet, daß** die Reaktionsdauer der Hydroxymethylierung zwischen 15 Minuten und 4 Stunden, vorzugsweise zwischen 1 Stunde und 3 Stunden, liegt.

38. Verfahren nach Anspruch 29, **dadurch gekennzeichnet, daß** man in die Apparatur Phenol, Formaldehyd und gegebenenfalls eine Base gibt, dann das Reaktionsmilieu unter Rühren für die Vervollständigung der Reaktion notwendige Dauer auf die gewünschte Temperatur bringt, um eine Mischung aus Phenolverbindungen der Formel (IIa₁) bis (IIc₁) zu erhalten.

39. Verfahren nach Anspruch 27, **dadurch gekennzeichnet, daß** eine Mischung der Verbindungen der Formeln (IIa₂) bis (IIc₂) durch Oxidation der hydroxymethylierten Verbindungen der Formel (IIa₁) bis (IIc₃) mittels molekularem Sauerstoff oder einem sauerstoffhaltigen Gas in wäßriger alkalischer Phase in Gegenwart eines Katalysators auf Basis eines Metalls der 8. Gruppe des Periodensystems der Elemente, vorzugsweise Platin oder Palladium, erhalten wird, wobei der Katalysator gegebenenfalls als Aktivator Metalle wie Cadmium, Cer, Bismut, Blei, Silber, Tellur oder Zinn umfaßt.

40. Verfahren nach Anspruch 39, **dadurch gekennzeichnet, daß** der pH-Wert der Lösung, gegebenenfalls durch Zugabe einer alkalischen oder erdalkalischen Base, auf Werte zwischen 8 und 13 gebracht wird.

41. Verfahren nach Anspruch 39, **dadurch gekennzeichnet, daß** die Reaktionstemperatur der Oxidation zwischen 10 °C und 100 °C, vorzugsweise zwischen 20 °C und 60°C, liegt.

42. Verfahren nach Anspruch 27, **dadurch gekennzeichnet, daß** eine Mischung der Phenolverbindungen der Formel (IIa₂) bis (IIc₂) durch ein zweistufiges Verfahren erhalten wird, das umfaßt:
- die Hydroxymethylierung eines Phenols in wäßrigem Milieu in Gegenwart einer alkalischen oder erdalkalischen Base durch Formaldehyd oder einen Formaldehyd-Erzeuger, welche zu einer Mischung aus hydroxymethylierten Phenolverbindungen führt, wobei eine Phenolverbindung in 2- und 4-Position hydroxymethyliert wird und die anderen beiden Phenolverbindungen in 2- oder 4-Position hydroxymethyliert werden,
- die Oxidation der erhaltenen Phenolverbindungen, ohne Isolierung der Zwischenprodukte, durch molekularem Sauerstoff oder ein sauerstoffhaltiges Gas in wäßriger alkalischer Phase in Gegenwart eines Katalysators auf Basis eines Metalls der 8. Gruppe des Periodensystems der Elemente, der gegebenenfalls als Aktivator ein wie zuvor beschriebenes Metall umfaßt.

43. Verfahren zur Herstellung von Vanillin gemäß einem der Ansprüche 1 bis 41, **dadurch gekennzeichnet, daß** man eine Mischung von 4,6-Di(hydroxymethyl)guajakol (A), p-Hydroxymethylguajakol (B) und o-Hydroxymethylguajakol (C) einer selektiven Oxidation der Hydroxymethylgruppe in 2-Position der Verbindungen (A) und (C) zu einer Carboxylgruppe und der Hydroxymethylgruppe in 4-Position der Verbindungen (A) und (B) zu einer Formylgruppe unterzieht, was zu einer Mischung von 3-Carboxy-4-hydroxy-5-methoxybenzaldehyd, Vanillin und 2-Hydroxy-3-methoxybenzoesäure führt, die nach Durchführung einer Decarboxylierung zu Vanillin und Guajakol, das rezykliert werden kann, führt.

44. Verfahren zur Herstellung von Vanillin gemäß einem der Ansprüche 1 bis 42, **dadurch gekennzeichnet, daß** man eine Mischung aus 4,6-Di(formyl)-guajakol (A), p-Formylguajakol (B) und o-Formylguajakol (C) einer selektiven Oxidation der Formylgruppe in 2-Position der Verbindungen (A) und (C) zu einer Carboxylgruppe unterzieht, was zu einer Mischung aus 3-Carboxy-4-hydroxy-5-methoxybenzaldehyd, Vanillin und 2-Hydroxy-3-methoxybenzoesäure führt, die nach Durchführung einer Decarboxylierung zu Vanillin und Guajakol, das rezykliert werden kann, führt.

45. Verfahren zur Herstellung von Ethylvanillin gemäß einem der Ansprüche 1 bis 41, **dadurch gekennzeichnet, daß** man eine Mischung aus 4,6-Di(hydroxymethyl)guetol (A), p-Hydroxymethylguetol (B) und o-Hydroxymethylguetol (C) einer selektiven Oxidation der Hydroxymethylgruppe in 2-Position der Verbindungen (A) und (C) zu einer Carboxylgruppe und der Hydroxymethylgruppe in 4-Position der Verbindungen (A) und (B) zu einer Formylgruppe unterzieht, was zu einer Mischung aus 3-Carboxy-4-hydroxy-5-ethoxybenzaldehyd, Ethylvanillin und 2-Hydroxy-3-ethoxybenzoesäure führt, die nach Durchführung einer Decarboxylierung zu Ethylvanillin und Guetol, das rezykliert werden kann, führt.

46. Verfahren zur Herstellung von Ethylanillin gemäß einem der Ansprüche 1 bis 45, **dadurch gekennzeichnet, daß** man eine Mischung aus 4,6-Di(formyl)guetol (A), p-Formylguetol (B) und o-Formylguetol (C) einer selektiven Oxidation der Formylgruppe in 2-Position der Verbindungen (A) und (C) zu einer Carboxylgruppe unterzieht, was zu einer Mischung aus 3-Carboxy-4-hydroxy-5-ethoxybenzaldehyd, Ethylvanillin und 2-Hydroxy-3-ethoxybenzoesäure führt, die nach Durchführung einer Decarboxylierung zu Ethylvanillin und Guetol, das rezykliert werden kann, führt.

47. Anwendung des Verfahrens, beschrieben in einem der Ansprüche 1 bis 46, zur Herstellung von Vanillin und Ethylvanillin.

## Claims

1. A process for preparing a 4-hydroxybenzaldehyde and its derivatives,
**characterized in that** it consists of selectively oxidising the group in the 2 position with respect to the hydroxyl group to a carboxy group, and optionally the hydroxymethyl group in the 4 position to a formyl group, present in the phenolic compounds in a mixture comprising at least:
• a phenolic compound (A) carrying formyl and/or hydroxymethyl groups in the 2 and 4 position;
• a phenolic compound (B) carrying a formyl or hydroxymethyl group in the 4 position;
• a phenolic compound (C) carrying a formyl or hydroxymethyl group in the 2 position;
resulting in a mixture comprising a 3-carboxy-4-hydroxybenzaldehyde, a 4-hydroxybenzaldehyde and a 2-hydroxybenzoic acid, which then undergoes a decarboxylation operation to produce the 4-hydroxybenzaldehyde and a phenol which can optionally be recycled.

2. A process for preparing a 4-hydroxybenzaldehyde and its derivatives, **characterized in that** a mixture comprising at least:
• a phenolic compound (A) carrying formyl and/or hydroxymethyl groups in the 2 and 4 position;
• a phenolic compound (B) carrying a formyl or hydroxymethyl group in the 4 position;
• a phenolic compound (C) carrying a formyl or hydroxymethyl group in the 2 position;
is selectively oxidised at the group in the 2 position to a carboxy group, and optionally the hydroxymethyl group in the 4 position to a formyl group, in the presence of a base and a catalyst based on a metal M1 selected from 1b and 8 metals, resulting in a mixture comprising a 3-carboxy-4-hydroxybenzaldehyde, a 4-hydroxybenzaldehyde and a 2-hydroxybenzoic acid, which then undergoes a decarboxylation operation by heating to produce the 4-hydroxybenzaldehyde and a phenol which can optionally be recycled.

3. A process according to claim 1 or claim 2, **characterized in that** the mixture of phenolic compounds has general formula (II): 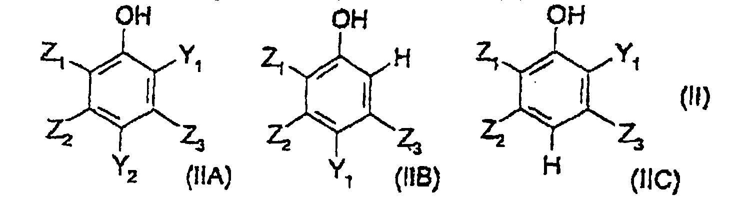 in which, in formulae (IIA) to (IIC):
• Y₁ and Y₂, which may be identical or different, represent one of the following groups:
• a -CHO group;
• a -CH₂OH group;
• Z₁, Z₂ and Z₃, which may be identical or different, represent a hydrogen atom, an alkyl, alkenyl, alkoxy, hydroxyalkyl, alkoxyalkyl, cycloalkyl or aryl radical, a hydroxy group, a nitro group, a halogen atom, or a trifluoromethyl group.

4. A process according to one of claims 1 to 3, **characterized in that** the phenolic compounds have formulae (IIA) to (IIC) where Z₁, Z₂ and Z₃, which may be identical or different, represent one of the following groups:
• a hydrogen atom;
• a linear or branched alkyl radical containing 1 to 12 carbon atoms, preferably 1 to 4 carbon atoms, such as methyl, ethyl, propyl, isopropyl, butyl, isobutyl, sec-butyl or tert-butyl;
• a linear or branched alkenyl radical containing 2 to 12 carbon atoms, preferably 2 to 4 carbon atoms, such as vinyl or allyl;
• a linear or branched alkoxy radical containing 1 to 12 carbon atoms, preferably 1 to 4 carbon atoms, such as a methoxy, ethoxy, propoxy, isopropoxy, butoxy, isobutoxy, sec-butoxy or tert-butoxy radical;
• a phenyl radical;
• a halogen atom, preferably a fluorine, chlorine or bromine atom.

5. A process according to one of claims I to 4, **characterized in that** the phenolic compounds have formulae (IIA) to (IIC) where Z₁ represents a hydrogen atom, or a linear or branched alkyl or alkoxy radical containing 1 to 6 carbon atoms, preferably 1 to 4 carbon atoms; Z₂ and Z₃ represent a hydrogen atom; and Y₁ and Y₂ are identical and represent a formyl group or a hydroxymethyl group.

6. A process according to one of claims I to 5, **characterized in that** the mixture of phenolic compounds with formula (II) is:
• o-hydroxymethylguaiacol, p-hydroxymethylguaiacol and 4,6-di(hydroxymethyl)guaiacol;
• o-formylguaiacol, p-formylguaiacol and 4,6-diformylguaiacol;
• o-hydroxymethylguetol, p-hydroxymethylguetol and 4,6-di(hydroxymethyl)guetol;
• o-formylguetol, p-formylguetol and 4,6-diformylguetol.

7. A process according to one of claims 1 to 6, **characterized in that** a mixture of phenolic compounds with formula (II) is oxidised in the liquid phase using molecular oxygen or a gas containing molecular oxygen, in an aqueous medium comprising a basic agent, in the presence of a catalyst based on a metal M₁ selected from metals from group 1b and 8 of the periodic classification of the elements, which catalyst may contain, as an activator, metals such as cadmium, cerium, bismuth, lead, silver, tellurium or tin.

8. A process according to claim 7, **characterized in that** the catalyst is based on copper, nickel, ruthenium, rhodium, palladium, osmium, iridium, platinum and mixtures thereof, said catalyst preferably being based on platinum and/or palladium.

9. A process according to claim 7 or claim 8, **characterized in that** the platinum and/or palladium based catalyst is provided in the form of platinum black, palladium black, platinum oxide, palladium oxide or the noble metal itself deposited on different supports such as carbon black, calcium carbonate, aluminas or activated silicas or equivalent materials, preferably carbon black.

10. A process according to any one of claims 7 to 9, **characterized in that** the quantity of catalyst used, expressed as the weight of metal M₁ with respect to that of the phenolic compound with formula (II), can vary from 0.01% to 10%, preferably 0.04% to 2%.

11. A process according to one of claims 7 to 10, **characterized in that** the activator is an organic or inorganic bismuth derivative selected from the group formed by: bismuth oxides; bismuth hydroxides; bismuth or bismuthyl salts of inorganic hydracids, preferably the chloride, bromide, iodide, sulphide, selenide or telluride; bismuth or bismuthyl salts of inorganic oxyacids, preferably the sulphite, sulphate, nitrite, nitrate, phosphite, phosphate, pyrophosphate, carbonate, perchlorate, antimonate, arsenate, selenite, or selenate; bismuth or bismuthyl salts of aliphatic or aromatic organic acids, preferably the acetate, propionate, salicylate, benzoate, oxalate, tartrate, lactate, or citrate; and bismuth or bismuthyl phenates, preferably the gallate or pyrogallate.

12. A process according to claim 11, **characterized in that** the bismuth derivative is selected from the group formed by: bismuth oxides Bi₂O₃ and Bi₂O₄; bismuth hydroxide Bi(OH)₃; bismuth chloride BiCl₃; bismuth bromide BiBr₃; bismuth iodide BiI₃; neutral bismuth sulphate Bi₂(SO₄)₃; neutral bismuth nitrate Bi(NO₃)₃,5H₂O; bismuthyl nitrate BiO(NO₃); bismuthyl carbonate (BiO)₂CO₃,0.5H₂O; bismuth acetate Bi(C₂H₃O₂)₃ and bismuth salicylate C₆H₄CO₂(BiO)OH.

13. A process according to one of claims 7 to 12, **characterized in that** the quantity of activator is selected so that the medium contains: at least 0.1% by weight of metal activator with respect to the weight of metal M₁ used, and 10 to 900 ppm by weight of metal M₁ with respect to the mixture of phenolic compounds with formula (II).

14. A process according to any one of claims 7 to 13, **characterized in that** the oxidation reaction is carried out within a temperature range of 30°C to 200°C, preferably between 40°C and 160°C.

15. A process according to any one of claims 7 to 14, **characterized in that** a pressure of 1 to 20 bar is used.

16. A process according to any one of claims 7 to 15, **characterized in that** oxidation is carried out in an aqueous medium containing, in solution, a basic agent, preferably sodium or potassium hydroxide, in a quantity such that it represents 0.5 to 10 moles, preferably 1 to 4 moles, more preferably 2 to 4 moles, of inorganic base per mole of phenolic compounds with formula (II).

17. A process according to claim 14, **characterized in that** when a platinum catalyst is used, the temperature is between 60°C and 160°C; the quantity of base used is in the range 1 to 3 moles per mole of phenolic compounds with formula (II).

18. A process according to claim 14, **characterized in that** when a palladium catalyst is used, the temperature is between 30°C and 200°C, preferably between 30°C and 150°C; the quantity of base used is in the range 2 to 4 moles per mole of phenolic compounds with formula (II).

19. A process according to any one of claims 1 to 18, **characterized in that** the reaction medium obtained comprising a 3-carboxy-4-hydroxybenzaldehyde, a 2-hydroxybenzoic acid and a 4-hydroxybenzaldehyde, completely or partially in their salt form, undergoes decarboxylation.

20. A process according to claim 19, **characterized in that** the acids to be decarboxylated have the following formula: 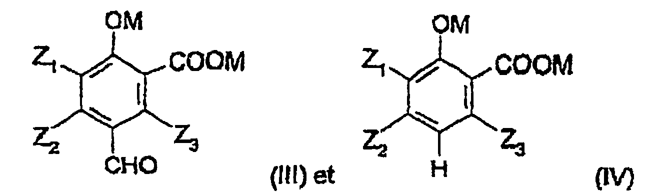 where, in said formulae (III) and (IV):
• M represents a hydrogen atom and/or a metallic cation from group (Ia) or (IIa), or an ammonium cation;
• Z₁, Z₂ and Z₃ have the meanings given in claims 3 to 5.

21. A process according to claim 20, **characterized in that** said acid is decarboxylated by adding to the reaction medium a protonic acid of inorganic origin, preferably hydrochloric acid or sulphuric acid or an organic acid, until a pH of no more than 3 is obtained.

22. A process according to any one of claims 20 to 22, **characterized in that** the reaction medium is heated to a temperature between 120°C and 350°C, preferably 150°C to 220°C and after cooling, the 4-hydroxybenzaldehyde is separated which preferably has the following formula (VI): 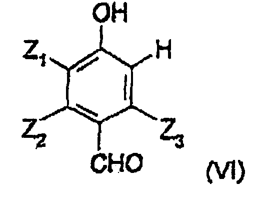 in which formula (VI):
• Z₁, Z₂ and Z₃ have the meanings given in claims 3 to 5.

23. A reaction medium obtained comprising a 3-carboxy-4-hydroxybenzaldehyde, a 2-hydroxybenzoic acid and a 4-hydroxybenzaldehyde, completely or partially in their salt form.

24. A process for preparing the mixture defined in claim 23, **characterized in that** it consists of selectively oxidising, in accordance with the process defined in one of claims 1 to 17, the group in the 2 position with respect to the hydroxyl group to a carboxy group, and optionally the hydroxymethyl group in the 4 position to a formyl group, present in the phenolic compounds in a mixture comprising at least:
• a phenolic compound (A) carrying formyl and/or hydroxymethyl groups in the 2 and 4 position;
• a phenolic compound (B) carrying a formyl or hydroxymethyl group in the 4 position;
• a phenolic compound (B) carrying a formyl or hydroxymethyl group in the 4 position;
• a phenolic compound (C) carrying a formyl or hydroxymethyl group in the 2 position.

25. A mixture of phenolic compounds which are completely or partially in their salt form, comprising:
• a phenolic compound (A) carrying formyl and/or hydroxymethyl groups in the 2 and 4 position;
• a phenolic compound (B) carrying a formyl group or a hydroxymethyl group in the 4 position;
• a phenolic compound (C) carrying a formyl or a hydroxymethyl group in the 2 position.

26. A mixture of phenolic compounds having general formula (II): 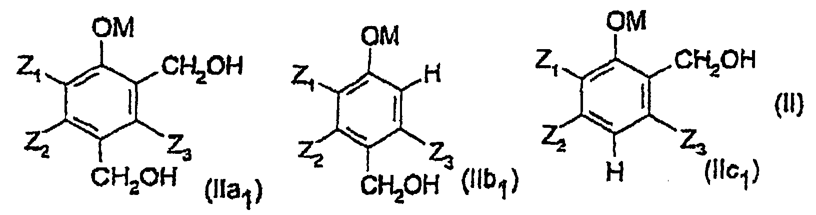 in which formulae:
• M represents a hydrogen atom and/or a metallic cation from group (Ia) or (IIa), or an ammonium cation;
• Z₁, Z₂ and Z₃ have the meanings given in claims 3 to 5.

27. A mixture of phenolic compounds having general formula (II'): 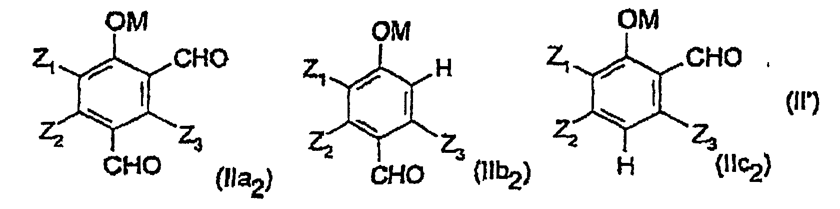 in which formulae:
• M represents a hydrogen atom and/or a metallic cation from group (Ia) or (IIa), or an ammonium cation;
• Z₁, Z₂ and Z₃ have the meanings given in claims 3 to 5.

28. A mixture of phenolic compounds according to any one of claims 24 to 26, **characterized in that** it comprises:
• 30% to 70% by weight, more preferably 50% to 70%, of a phenolic compound (A);
• 30% to 70%, more preferably 50% to 70%, of a mixture of phenolic compounds (B+C)..

29. A process for preparing a mixture of phenolic compounds described in any one of claims 25 to 28, **characterized in that** it is obtained by hydroxymethylating a phenol by condensing it with formaldehyde or a formaldehyde generator in an aqueous phase in the presence of an alkaline or alkaline-earth base such that the degree of phenol conversion is at most 95%, optionally followed by an oxidation step.

30. A process according to claim 29, **characterized in that** the degree of phenol conversion is 60% to 95%, preferably 80% to 95%.

31. A process according to claim 29 or claim 30, **characterized in that** the starting phenol is a phenol which is not substituted in the ortho and para positions with respect to the hydroxyl group, with general formula (I): 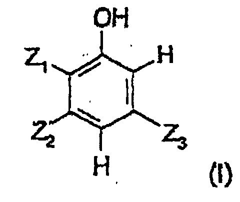 where Z₁, Z₂ and Z₃ have the meanings given above.

32. A process according to claim 31, **characterized in that** the phenol with formula (I) is guaiacol, guetol, 3-methoxyphenol, 3-ethoxyphenol, 3-isopropoxyphenol, 3-t-butoxyphenol, m-cresol, or o-cresol.

33. A process according to any one of claims 29 to 32, **characterized in that** formaldehyde or any formaldehyde generator is used, preferably trioxane or para-formaldehyde, used in the form of linear polyformaldehydes with any degree of polymerisation, preferably containing 8 to 100 (CH₂O) units.

34. A process according to claim 33. **characterized in that** the formaldehyde/phenol molar ratio is between 1.0 and 4.0, preferably between 1.0 and 2.5.

35. A process according to claim 29, **characterized in that** the quantity of base present in the hydroxymethylation medium, expressed as the number of moles of base/ phenolic hydroxy group of the phenol to be hydroxymethylated, is between 1.0 and 4.0, preferably between 0.9 and 2.0.

36. A process according to claim 29, **characterized in that** the hydroxymethylation temperature is in the range 0°C to 100°C, preferably in the range 20°C to 70°C.

37. A process according to claim 29, **characterized in that** the duration of the hydroxymethylation reaction is between 15 minutes and 4 hours, preferably between 1 hour and 3 hours.

38. A process according to claim 29, **characterized in that** the phenol and the formaldehyde and the optional base are charged into the apparatus, then the reaction mixture is heated to the desired temperature with stirring, for the period necessary to carry out the reaction until a mixture of phenolic compounds with formula (IIa₁) to (IIc₁) is obtained.

39. A process according to claim 27, **characterized in that** a mixture of compounds with formula (IIa₂) (IIc₂) is prepared by oxidising hydroxymethylated phenolic compounds with formula (IIa₁) to (IIc₁), by oxidising using molecular oxygen or a gas containing molecular oxygen, in an aqueous alkaline phase in the presence of a catalyst based on a metal from group 8 of the periodic table, preferably platinum or palladium, and optionally containing metals such as cadmium, cerium, bismuth, lead, silver, tellurium or tin as an activator.

40. A process according to claim 39, **characterized in that** the pH of the solution is brought to a value in the range 8 to 13 by optionally adding an alkaline or alkaline-earth base.

41. A process according to claim 39, **characterized in that** the temperature of the oxidation reaction is in the range 10°C to 100°C, preferably in the range 20°C to 60°C.

42. A process according to claim 27, **characterized in that** a mixture of phenolic compounds with formula (IIa₂) to (IIc₂) is obtained by a two-step process comprising:
• hydroxymethylation of a phenol in an aqueous medium in the presence of an alkaline or alkaline-earth base using formaldehyde or a formaldehyde generator resulting in a mixture of hydroxymethylated phenolic compounds, one being hydroxymethylated in the 2 and 4 positions and the two others being hydroxymethylated in the 2 or in the 4 position;
• and oxidation, without intermediate separation, of the phenolic compounds obtained using molecular oxygen or a gas containing molecular oxygen, in an aqueous alkaline phase in the presence of a catalyst based on a metal from group 8 of the periodic table, and optionally, a metal such as those cited above, acting as an activator.

43. A process for preparing vanillin according to any one of claims 1 to 41, **characterized in that** a mixture of phenolic compounds, 4,6-di(hydroxymethyl)guaiacol (A), p-hydroxymethylguaiacol (B) and o-hydroxymethylguaiacol (C) is selectively oxidised at the hydroxymethyl group in the 2-position of compounds (A) and (C) to a carboxy group, and at the hydroxymethyl group in the 4 position in compounds (A) and (B) to a formyl group, resulting in a mixture of 3-carboxy-4-hydroxy-5-methoxybenzaldehyde, vanillin and 2-hydroxy-3-methoxybenzoic acid which, after decarboxylation, produces vanillin and guaiacol which can be recycled.

44. A process for the preparation of vanillin according to any one of claims 1 to 42, **characterized in that** a mixture of phenolic compounds, 4,6-di(formyl)guaiacol (A), p-formylguaiacol (B) and o-formylguaiacol (C) is selectively oxidised at the formyl group in the 2-position of compounds (A) and (C) to a carboxy group, resulting in a mixture of 3-carboxy-4-hydroxy-5-methoxybenzaldehyde, vanillin and 2-hydroxy-3-methoxybenzoic acid which, after decarboxylation, produces vanillin and guaiacol which can be recycled.

45. A process for the preparation of ethylvanillin according to any one of claims I to 41, **characterized in that** a mixture of phenolic compounds, 4,6-di(hydroxymethyl)guetol (A), p-hydroxymethylguetol (B) and o-hydroxymethylguetol (C) is selectively oxidised at the hydroxymethyl group in the 2-position of compounds (A) and (C) to a carboxy group, and at the hydroxymethyl group in the 4 position in compounds (A) and (B) to a formyl group, resulting in a mixture of 3-carboxy-4-hydroxy-5-ethoxybenzaldehyde, ethylvanillin and 2-hydroxy-3-ethoxybenzoic acid which, after decarboxylation, produces ethylvanillin and guetol which can be recycled.

46. A process for the preparation of ethylvanillin according to any one of claims 1 to 45, **characterized in that** a mixture of phenolic compounds, 4,6-di(formyl)guetol (A), p-formylguetol (B) and o-formylguetol (C) is selectively oxidised at the formyl group in the 2-position of compounds (A) and (C) to a carboxy group, resulting in a mixture of 3-carboxy-4-hydroxy-5-ethoxybenzaldehyde, ethylvanillin and 2-hydroxy-3-ethoxybenzoic acid which, after decarboxylation, produces ethylvanillin and guetol which can be recycled.

47. Use of the process described in any one of claims I to 46 for the preparation of vanillin and ethylvanillin.
